(19) Europäisches Patentamt
European Patent Office
Office européen des brevets

(11) EP 4 183 875 A1

(12) EUROPEAN PATENT APPLICATION

(43) Date of publication:
**24.05.2023 Bulletin 2023/21**

(21) Application number: **21209450.2**

(22) Date of filing: **22.11.2021**

(51) International Patent Classification (IPC):
***C12N 9/02*** *(2006.01)* ***C12N 9/10*** *(2006.01)*
***C12N 15/63*** *(2006.01)*

(52) Cooperative Patent Classification (CPC):
**C12N 15/70; C12N 9/0028; C12N 9/0071; C12Y 114/14003;** C12N 15/74; C12Q 1/04; C12Q 1/66; C12R 2001/01; C12R 2001/19; C12Y 105/01038

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.**
**80539 München (DE)**

(72) Inventor: **GREGOR, Carola**
**37073 Göttingen (DE)**

(74) Representative: **Günther, Constantin et al**
**Meissner Bolte Patentanwälte**
**Rechtsanwälte Partnerschaft mbB**
**Plathnerstraße 3A**
**30175 Hannover (DE)**

Remarks:
The complete document including Reference Table(s) and the Sequence Listing(s) can be downloaded from the EPO website

# (54) IMPROVED BIOLUMINESCENT SYSTEM

(57) The present invention relates in a first aspect to an isolated nucleic acid molecule, comprising at least nucleic acid sequences encoding the luxC polypeptide with specific mutations compared to the wild type sequence as well as nucleic acid sequences encoding the luxD polypeptide containing amino acid substitutions as well as nucleotide derivatives thereof. Moreover, the polypeptides encoded are disclosed as well as operons, vectors and bacteriophages containing said nucleic acid sequences. In addition, cells in particular bacterial cells are provided containing the mutated luxC and luxD genes encoding the specific luxC and luxD polypeptides according to the present invention. Moreover, a method of generation of bright autobioluminescent cells is provided as well as kits comprising at least one isolated nucleic acid molecule according to the present invention or operons or vectors or bacteriophages. Finally, the use of molecules according to present inventions, proteins, operons, vectors and bacteriophages or cells in a process of bioluminescence imaging in living cells is provided.

EP 4 183 875 A1

SEQUENCE LISTING

<110> Max-Planck-Gesellschaft zur Foerderung der Wissenschaften e.V.

<120> improved bioluminescent system

<130> 4910-0013 EP-1

<160> 29

<170> PatentIn version 3.5

<210> 1
<211> 1443
<212> DNA
<213> P. luminescens

<220>
<221> CDS
<222> (1)..(1443)

<400> 1
```
atg act aaa aaa att tca ttc att att aac ggc cag gtt gaa atc ttt        48
Met Thr Lys Lys Ile Ser Phe Ile Ile Asn Gly Gln Val Glu Ile Phe
1               5                   10                  15

ccc gaa agt gat gat tta gtg caa tcc att aat ttt ggt gat aat agt        96
Pro Glu Ser Asp Asp Leu Val Gln Ser Ile Asn Phe Gly Asp Asn Ser
            20                  25                  30

gtt tac ctg cca ata ttg aat gac tct cat gta aaa aac att att gat       144
Val Tyr Leu Pro Ile Leu Asn Asp Ser His Val Lys Asn Ile Ile Asp
        35                  40                  45

tgt aat gga aat aac gaa tta cgg ttg cat aac att gtc aat ttt ctc       192
Cys Asn Gly Asn Asn Glu Leu Arg Leu His Asn Ile Val Asn Phe Leu
    50                  55                  60

tat acg gta ggg caa aga tgg aaa aat gaa gaa tac tca aga cgc agg       240
Tyr Thr Val Gly Gln Arg Trp Lys Asn Glu Glu Tyr Ser Arg Arg Arg
65                  70                  75                  80

aca tac att cgt gac tta aaa aaa tat atg gga tat tca gaa gaa atg       288
Thr Tyr Ile Arg Asp Leu Lys Lys Tyr Met Gly Tyr Ser Glu Glu Met
                85                  90                  95

gct aag cta gag gcc aat tgg ata tct atg att tta tgt tct aaa ggc       336
Ala Lys Leu Glu Ala Asn Trp Ile Ser Met Ile Leu Cys Ser Lys Gly
            100                 105                 110

ggc ctt tat gat gtt gta gaa aat gaa ctt ggt tct cgc cat atc atg       384
Gly Leu Tyr Asp Val Val Glu Asn Glu Leu Gly Ser Arg His Ile Met
        115                 120                 125

gat gaa tgg cta cct cag gat gaa agt tat gtt cgg gct ttt ccg aaa       432
Asp Glu Trp Leu Pro Gln Asp Glu Ser Tyr Val Arg Ala Phe Pro Lys
    130                 135                 140

ggt aaa tct gta cat ctg ttg gca ggt aat gtt cca tta tct ggg atc       480
Gly Lys Ser Val His Leu Leu Ala Gly Asn Val Pro Leu Ser Gly Ile
145                 150                 155                 160
```

atg tct ata tta cgc gca att tta act aag aat cag tgt att ata aaa 528
Met Ser Ile Leu Arg Ala Ile Leu Thr Lys Asn Gln Cys Ile Ile Lys
165 170 175

aca tcg tca acc gat cct ttt acc gct aat gca tta gcg tta agt ttt 576
Thr Ser Ser Thr Asp Pro Phe Thr Ala Asn Ala Leu Ala Leu Ser Phe
180 185 190

att gat gta gac cct aat cat ccg ata acg cgc tct tta tct gtt ata 624
Ile Asp Val Asp Pro Asn His Pro Ile Thr Arg Ser Leu Ser Val Ile
195 200 205

tat tgg ccc cac caa ggt gat aca tca ctc gca aaa gaa att atg cga 672
Tyr Trp Pro His Gln Gly Asp Thr Ser Leu Ala Lys Glu Ile Met Arg
210 215 220

cat gcg gat gtt att gtc gct tgg gga ggg cca gat gcg att aat tgg 720
His Ala Asp Val Ile Val Ala Trp Gly Gly Pro Asp Ala Ile Asn Trp
225 230 235 240

gcg gta gag cat gcg cca tct tat gct gat gtg att aaa ttt ggt tct 768
Ala Val Glu His Ala Pro Ser Tyr Ala Asp Val Ile Lys Phe Gly Ser
245 250 255

aaa aag agt ctt tgc att atc gat aat cct gtt gat ttg acg tcc gca 816
Lys Lys Ser Leu Cys Ile Ile Asp Asn Pro Val Asp Leu Thr Ser Ala
260 265 270

gcg aca ggt gcg gct cat gat gtt tgt ttt tac gat cag cga gct tgt 864
Ala Thr Gly Ala Ala His Asp Val Cys Phe Tyr Asp Gln Arg Ala Cys
275 280 285

ttt tct gcc caa aac ata tat tac atg gga aat cat tat gag gaa ttt 912
Phe Ser Ala Gln Asn Ile Tyr Tyr Met Gly Asn His Tyr Glu Glu Phe
290 295 300

aag tta gcg ttg ata gaa aaa ctt aat cta tat gcg cat ata tta ccg 960
Lys Leu Ala Leu Ile Glu Lys Leu Asn Leu Tyr Ala His Ile Leu Pro
305 310 315 320

aat gcc aaa aaa gat ttt gat gaa aag gcg gcc tat tct tta gtt caa 1008
Asn Ala Lys Lys Asp Phe Asp Glu Lys Ala Ala Tyr Ser Leu Val Gln
325 330 335

aaa gaa agc ttg ttt gct gga tta aaa gta gag gtg gat att cat caa 1056
Lys Glu Ser Leu Phe Ala Gly Leu Lys Val Glu Val Asp Ile His Gln
340 345 350

cgt tgg atg att att gag tca aat gca ggt gtg gaa ttt aat caa cca 1104
Arg Trp Met Ile Ile Glu Ser Asn Ala Gly Val Glu Phe Asn Gln Pro
355 360 365

ctt ggc aga tgt gtg tac ctt cat cac gtc gat aat att gag caa ata 1152
Leu Gly Arg Cys Val Tyr Leu His His Val Asp Asn Ile Glu Gln Ile
370 375 380

ttg cct tat gtt caa aaa aat aag acg caa acc ata tct att ttt cct 1200
Leu Pro Tyr Val Gln Lys Asn Lys Thr Gln Thr Ile Ser Ile Phe Pro
385 390 395 400

tgg gag tca tca ttt aaa tat cga gat gcg tta gca tta aaa ggt gcg 1248
Trp Glu Ser Ser Phe Lys Tyr Arg Asp Ala Leu Ala Leu Lys Gly Ala

405 410 415

gaa agg att gta gaa gca gga atg aat aac ata ttt cga gtt ggt gga 1296
Glu Arg Ile Val Glu Ala Gly Met Asn Asn Ile Phe Arg Val Gly Gly
420 425 430

tct cat gac gga atg aga ccg ttg caa cga tta gtg aca tat att tct 1344
Ser His Asp Gly Met Arg Pro Leu Gln Arg Leu Val Thr Tyr Ile Ser
435 440 445

cat gaa agg cca tct aac tat acg gct aag gat gtt gcg gtt gaa ata 1392
His Glu Arg Pro Ser Asn Tyr Thr Ala Lys Asp Val Ala Val Glu Ile
450 455 460

gaa cag act cga ttc ctg gaa gaa gat aag ttc ctt gta ttt gtc cca 1440
Glu Gln Thr Arg Phe Leu Glu Glu Asp Lys Phe Leu Val Phe Val Pro
465 470 475 480

taa 1443

<210> 2
<211> 480
<212> PRT
<213> P. luminescens

<400> 2

Met Thr Lys Lys Ile Ser Phe Ile Ile Asn Gly Gln Val Glu Ile Phe
1 5 10 15

Pro Glu Ser Asp Asp Leu Val Gln Ser Ile Asn Phe Gly Asp Asn Ser
20 25 30

Val Tyr Leu Pro Ile Leu Asn Asp Ser His Val Lys Asn Ile Ile Asp
35 40 45

Cys Asn Gly Asn Asn Glu Leu Arg Leu His Asn Ile Val Asn Phe Leu
50 55 60

Tyr Thr Val Gly Gln Arg Trp Lys Asn Glu Glu Tyr Ser Arg Arg Arg
65 70 75 80

Thr Tyr Ile Arg Asp Leu Lys Lys Tyr Met Gly Tyr Ser Glu Glu Met
85 90 95

Ala Lys Leu Glu Ala Asn Trp Ile Ser Met Ile Leu Cys Ser Lys Gly
100 105 110

Gly Leu Tyr Asp Val Val Glu Asn Glu Leu Gly Ser Arg His Ile Met
115 120 125

Asp Glu Trp Leu Pro Gln Asp Glu Ser Tyr Val Arg Ala Phe Pro Lys
130 135 140

```
Gly Lys Ser Val His Leu Leu Ala Gly Asn Val Pro Leu Ser Gly Ile
145                 150                 155                 160

Met Ser Ile Leu Arg Ala Ile Leu Thr Lys Asn Gln Cys Ile Ile Lys
                165                 170                 175

Thr Ser Ser Thr Asp Pro Phe Thr Ala Asn Ala Leu Ala Leu Ser Phe
            180                 185                 190

Ile Asp Val Asp Pro Asn His Pro Ile Thr Arg Ser Leu Ser Val Ile
        195                 200                 205

Tyr Trp Pro His Gln Gly Asp Thr Ser Leu Ala Lys Glu Ile Met Arg
    210                 215                 220

His Ala Asp Val Ile Val Ala Trp Gly Gly Pro Asp Ala Ile Asn Trp
225                 230                 235                 240

Ala Val Glu His Ala Pro Ser Tyr Ala Asp Val Ile Lys Phe Gly Ser
                245                 250                 255

Lys Lys Ser Leu Cys Ile Ile Asp Asn Pro Val Asp Leu Thr Ser Ala
            260                 265                 270

Ala Thr Gly Ala Ala His Asp Val Cys Phe Tyr Asp Gln Arg Ala Cys
        275                 280                 285

Phe Ser Ala Gln Asn Ile Tyr Tyr Met Gly Asn His Tyr Glu Glu Phe
    290                 295                 300

Lys Leu Ala Leu Ile Glu Lys Leu Asn Leu Tyr Ala His Ile Leu Pro
305                 310                 315                 320

Asn Ala Lys Lys Asp Phe Asp Glu Lys Ala Ala Tyr Ser Leu Val Gln
                325                 330                 335

Lys Glu Ser Leu Phe Ala Gly Leu Lys Val Glu Val Asp Ile His Gln
            340                 345                 350

Arg Trp Met Ile Ile Glu Ser Asn Ala Gly Val Glu Phe Asn Gln Pro
        355                 360                 365

Leu Gly Arg Cys Val Tyr Leu His His Val Asp Asn Ile Glu Gln Ile
    370                 375                 380

Leu Pro Tyr Val Gln Lys Asn Lys Thr Gln Thr Ile Ser Ile Phe Pro
385                 390                 395                 400
```

Trp Glu Ser Ser Phe Lys Tyr Arg Asp Ala Leu Ala Leu Lys Gly Ala
405 410 415

Glu Arg Ile Val Glu Ala Gly Met Asn Asn Ile Phe Arg Val Gly Gly
420 425 430

Ser His Asp Gly Met Arg Pro Leu Gln Arg Leu Val Thr Tyr Ile Ser
435 440 445

His Glu Arg Pro Ser Asn Tyr Thr Ala Lys Asp Val Ala Val Glu Ile
450 455 460

Glu Gln Thr Arg Phe Leu Glu Glu Asp Lys Phe Leu Val Phe Val Pro
465 470 475 480

<210> 3
<211> 1443
<212> DNA
<213> artificial sedquence

<220>
<223> mutated LuxC from P. luminescens

<220>
<221> CDS
<222> (1)..(1443)

<400> 3
atg act aaa aaa att tca ttc att att acc ggc cag gtt gaa atc ttt 48
Met Thr Lys Lys Ile Ser Phe Ile Ile Thr Gly Gln Val Glu Ile Phe
1 5 10 15

ccc gaa agc gat gat tta gtg caa tcc att aat ttt ggt gat aat agt 96
Pro Glu Ser Asp Asp Leu Val Gln Ser Ile Asn Phe Gly Asp Asn Ser
20 25 30

gtt tac ctg cca ata ttg aat gac tct cat gta aaa aac att att gat 144
Val Tyr Leu Pro Ile Leu Asn Asp Ser His Val Lys Asn Ile Ile Asp
35 40 45

tgt aat gga aat aac gaa tta cgg ttg cat gac att gtc aat ttt ctc 192
Cys Asn Gly Asn Asn Glu Leu Arg Leu His Asp Ile Val Asn Phe Leu
50 55 60

tat acg gta ggg caa aga tgg aaa aat gat gaa tac tca aga cgc agg 240
Tyr Thr Val Gly Gln Arg Trp Lys Asn Asp Glu Tyr Ser Arg Arg Arg
65 70 75 80

aca tac att cgt gac tta aaa aaa tat atg gga tat tca gaa gaa atg 288
Thr Tyr Ile Arg Asp Leu Lys Lys Tyr Met Gly Tyr Ser Glu Glu Met
85 90 95

gct aag cta gag gcc aat tgg ata tct atg ata tta tgt tct aaa ggc 336
Ala Lys Leu Glu Ala Asn Trp Ile Ser Met Ile Leu Cys Ser Lys Gly
100 105 110

```
ggc ctt tat gat gtt gta gaa aat gaa ctt ggt tct cgc cat atc atg         384
Gly Leu Tyr Asp Val Val Glu Asn Glu Leu Gly Ser Arg His Ile Met
                115                 120                 125

gat gaa tgg cta cct cag gat gaa agt tat gtt cgg gct ttt ccg aaa         432
Asp Glu Trp Leu Pro Gln Asp Glu Ser Tyr Val Arg Ala Phe Pro Lys
    130                 135                 140

ggt aaa tct gta cat ctg ttg gca ggt aat gtt cca tta tct ggg atc         480
Gly Lys Ser Val His Leu Leu Ala Gly Asn Val Pro Leu Ser Gly Ile
145                 150                 155                 160

atg tct ata tta cgc gca att tta act aag aat cag tgt att ata aaa         528
Met Ser Ile Leu Arg Ala Ile Leu Thr Lys Asn Gln Cys Ile Ile Lys
                165                 170                 175

aca tcg tca acc gat cct ttt acc gct aat gca tta gcg tta agt ttt         576
Thr Ser Ser Thr Asp Pro Phe Thr Ala Asn Ala Leu Ala Leu Ser Phe
            180                 185                 190

att gat gta gac cct aat cat ccg ata acg cgc tct tta tct gtt ata         624
Ile Asp Val Asp Pro Asn His Pro Ile Thr Arg Ser Leu Ser Val Ile
        195                 200                 205

tat tgg ccc cac caa ggt gat aca tca ctc gca aaa gaa att atg cga         672
Tyr Trp Pro His Gln Gly Asp Thr Ser Leu Ala Lys Glu Ile Met Arg
    210                 215                 220

cat gcg gat gtt att gtc gct tgg gga ggg cca gat gcg att aat tgg         720
His Ala Asp Val Ile Val Ala Trp Gly Gly Pro Asp Ala Ile Asn Trp
225                 230                 235                 240

gcg gta gag cat gcg cca tct tat gct gat gtg att aaa ttt ggt cct         768
Ala Val Glu His Ala Pro Ser Tyr Ala Asp Val Ile Lys Phe Gly Pro
                245                 250                 255

aaa aag agt ctt tgc att atc gat aat cct gtt gat ttg acg tct gca         816
Lys Lys Ser Leu Cys Ile Ile Asp Asn Pro Val Asp Leu Thr Ser Ala
            260                 265                 270

gcg aca ggt gcg gct cat gat gtt tgt ttt tac gat cag cga gct tgt         864
Ala Thr Gly Ala Ala His Asp Val Cys Phe Tyr Asp Gln Arg Ala Cys
        275                 280                 285

ttt tct gcc caa aac ata tat tat atg gga aat cat tat gag gaa ttt         912
Phe Ser Ala Gln Asn Ile Tyr Tyr Met Gly Asn His Tyr Glu Glu Phe
    290                 295                 300

aag tta gcg ttg ata gaa aaa ctt aat cta tat gcg cat ata tta ccg         960
Lys Leu Ala Leu Ile Glu Lys Leu Asn Leu Tyr Ala His Ile Leu Pro
305                 310                 315                 320

aat gca aaa aaa gat ttt gat gaa aag gcg gcc tat tct tta gtt caa        1008
Asn Ala Lys Lys Asp Phe Asp Glu Lys Ala Ala Tyr Ser Leu Val Gln
                325                 330                 335

aaa gaa agc ttg ttt gct gga tta aaa gta gag gtg gat att cat caa        1056
Lys Glu Ser Leu Phe Ala Gly Leu Lys Val Glu Val Asp Ile His Gln
            340                 345                 350

cgt tgg acg att att gag tca gat gca ggt gtg gaa ttt aat caa cca        1104
Arg Trp Thr Ile Ile Glu Ser Asp Ala Gly Val Glu Phe Asn Gln Pro
```

```
        355                 360                 365

ctt ggc aga tgt gtg tac ctt cat cac gtc gat aat att gag caa ata        1152
Leu Gly Arg Cys Val Tyr Leu His His Val Asp Asn Ile Glu Gln Ile
    370                 375                 380

ttg cct tat gtt caa aaa aat aag acg caa acc ata tct att ttt cct        1200
Leu Pro Tyr Val Gln Lys Asn Lys Thr Gln Thr Ile Ser Ile Phe Pro
385                 390                 395                 400

tgg gag tca tca ttt aaa tat cga gat gcg tta gca tta aaa ggt gcg        1248
Trp Glu Ser Ser Phe Lys Tyr Arg Asp Ala Leu Ala Leu Lys Gly Ala
                405                 410                 415

gaa agg att gta gaa gca gga atg aat aac ata ttt cga gtt ggt gga        1296
Glu Arg Ile Val Glu Ala Gly Met Asn Asn Ile Phe Arg Val Gly Gly
            420                 425                 430

tct cat gac gga atg aga ccg ttg caa cga tta gtg aca tat att tct        1344
Ser His Asp Gly Met Arg Pro Leu Gln Arg Leu Val Thr Tyr Ile Ser
        435                 440                 445

cat gaa agg cca tct aac tat acg gct aag gat gtt gcg gtt gaa ata        1392
His Glu Arg Pro Ser Asn Tyr Thr Ala Lys Asp Val Ala Val Glu Ile
    450                 455                 460

gaa cag act cgt ttc ctg gaa gaa gat aag ttc ctt gta ttt gtc cca        1440
Glu Gln Thr Arg Phe Leu Glu Glu Asp Lys Phe Leu Val Phe Val Pro
465                 470                 475                 480

taa                                                                    1443


<210>  4
<211>  480
<212>  PRT
<213>  artificial sedquence

<220>
<223>  Synthetic Construct

<400>  4

Met Thr Lys Lys Ile Ser Phe Ile Ile Thr Gly Gln Val Glu Ile Phe
1               5                   10                  15


Pro Glu Ser Asp Asp Leu Val Gln Ser Ile Asn Phe Gly Asp Asn Ser
            20                  25                  30


Val Tyr Leu Pro Ile Leu Asn Asp Ser His Val Lys Asn Ile Ile Asp
        35                  40                  45


Cys Asn Gly Asn Asn Glu Leu Arg Leu His Asp Ile Val Asn Phe Leu
    50                  55                  60


Tyr Thr Val Gly Gln Arg Trp Lys Asn Asp Glu Tyr Ser Arg Arg Arg
65                  70                  75                  80
```

```
Thr Tyr Ile Arg Asp Leu Lys Lys Tyr Met Gly Tyr Ser Glu Glu Met
                85                  90                  95

Ala Lys Leu Glu Ala Asn Trp Ile Ser Met Ile Leu Cys Ser Lys Gly
            100                 105                 110

Gly Leu Tyr Asp Val Val Glu Asn Glu Leu Gly Ser Arg His Ile Met
        115                 120                 125

Asp Glu Trp Leu Pro Gln Asp Glu Ser Tyr Val Arg Ala Phe Pro Lys
    130                 135                 140

Gly Lys Ser Val His Leu Leu Ala Gly Asn Val Pro Leu Ser Gly Ile
145                 150                 155                 160

Met Ser Ile Leu Arg Ala Ile Leu Thr Lys Asn Gln Cys Ile Ile Lys
                165                 170                 175

Thr Ser Ser Thr Asp Pro Phe Thr Ala Asn Ala Leu Ala Leu Ser Phe
            180                 185                 190

Ile Asp Val Asp Pro Asn His Pro Ile Thr Arg Ser Leu Ser Val Ile
        195                 200                 205

Tyr Trp Pro His Gln Gly Asp Thr Ser Leu Ala Lys Glu Ile Met Arg
    210                 215                 220

His Ala Asp Val Ile Val Ala Trp Gly Gly Pro Asp Ala Ile Asn Trp
225                 230                 235                 240

Ala Val Glu His Ala Pro Ser Tyr Ala Asp Val Ile Lys Phe Gly Pro
                245                 250                 255

Lys Lys Ser Leu Cys Ile Ile Asp Asn Pro Val Asp Leu Thr Ser Ala
            260                 265                 270

Ala Thr Gly Ala Ala His Asp Val Cys Phe Tyr Asp Gln Arg Ala Cys
        275                 280                 285

Phe Ser Ala Gln Asn Ile Tyr Tyr Met Gly Asn His Tyr Glu Glu Phe
    290                 295                 300

Lys Leu Ala Leu Ile Glu Lys Leu Asn Leu Tyr Ala His Ile Leu Pro
305                 310                 315                 320

Asn Ala Lys Lys Asp Phe Asp Glu Lys Ala Ala Tyr Ser Leu Val Gln
                325                 330                 335
```

```
Lys Glu Ser Leu Phe Ala Gly Leu Lys Val Glu Val Asp Ile His Gln
            340                 345                 350

Arg Trp Thr Ile Ile Glu Ser Asp Ala Gly Val Glu Phe Asn Gln Pro
        355                 360                 365

Leu Gly Arg Cys Val Tyr Leu His His Val Asp Asn Ile Glu Gln Ile
    370                 375                 380

Leu Pro Tyr Val Gln Lys Asn Lys Thr Gln Thr Ile Ser Ile Phe Pro
385                 390                 395                 400

Trp Glu Ser Ser Phe Lys Tyr Arg Asp Ala Leu Ala Leu Lys Gly Ala
                405                 410                 415

Glu Arg Ile Val Glu Ala Gly Met Asn Asn Ile Phe Arg Val Gly Gly
            420                 425                 430

Ser His Asp Gly Met Arg Pro Leu Gln Arg Leu Val Thr Tyr Ile Ser
        435                 440                 445

His Glu Arg Pro Ser Asn Tyr Thr Ala Lys Asp Val Ala Val Glu Ile
    450                 455                 460

Glu Gln Thr Arg Phe Leu Glu Glu Asp Lys Phe Leu Val Phe Val Pro
465                 470                 475                 480


<210>  5
<211>  924
<212>  DNA
<213>  P. luminescens


<220>
<221>  CDS
<222>  (1)..(921)

<400>  5
atg gaa aat gaa tca aaa tat aaa acc atc gac cac gtt att tgt gtt        48
Met Glu Asn Glu Ser Lys Tyr Lys Thr Ile Asp His Val Ile Cys Val
1               5                   10                  15

gaa gga aat aaa aaa att cat gtt tgg gaa acg ctg cca gaa gaa aac        96
Glu Gly Asn Lys Lys Ile His Val Trp Glu Thr Leu Pro Glu Glu Asn
            20                  25                  30

agc cca aag aga aag aat gcc att att att gcg tct ggt ttt gcc cgc       144
Ser Pro Lys Arg Lys Asn Ala Ile Ile Ile Ala Ser Gly Phe Ala Arg
        35                  40                  45

agg atg gat cat ttt gct ggt ctg gcg gaa tat tta tcg cgg aat gga       192
Arg Met Asp His Phe Ala Gly Leu Ala Glu Tyr Leu Ser Arg Asn Gly
    50                  55                  60
```

```
ttt cat gtg atc cgc tat gat tcg ctt cac cac gtt gga ttg agt tca      240
Phe His Val Ile Arg Tyr Asp Ser Leu His His Val Gly Leu Ser Ser
65                  70                  75                  80

ggg aca att gat gaa ttt aca atg tct ata gga aag cag agc ttg tta      288
Gly Thr Ile Asp Glu Phe Thr Met Ser Ile Gly Lys Gln Ser Leu Leu
                85                  90                  95

gca gtg gtt gat tgg tta act aca cga aaa ata aat aac ttc ggt atg      336
Ala Val Val Asp Trp Leu Thr Thr Arg Lys Ile Asn Asn Phe Gly Met
            100                 105                 110

ttg gct tca agc tta tct gcg cgg ata gct tat gca agc cta tct gaa      384
Leu Ala Ser Ser Leu Ser Ala Arg Ile Ala Tyr Ala Ser Leu Ser Glu
        115                 120                 125

atc aat gct tcg ttt tta atc acc gca gtc ggt gtt gtt aac tta aga      432
Ile Asn Ala Ser Phe Leu Ile Thr Ala Val Gly Val Val Asn Leu Arg
    130                 135                 140

tat tct ctt gaa aga gct tta ggg ttt gat tat ctc agt cta ccc att      480
Tyr Ser Leu Glu Arg Ala Leu Gly Phe Asp Tyr Leu Ser Leu Pro Ile
145                 150                 155                 160

aat gaa ttg ccg aat aat cta gat ttt gaa ggc cat aaa ttg ggt gct      528
Asn Glu Leu Pro Asn Asn Leu Asp Phe Glu Gly His Lys Leu Gly Ala
                165                 170                 175

gaa gtc ttt gcg aga gat tgt ctt gat ttt ggt tgg gaa gat tta gct      576
Glu Val Phe Ala Arg Asp Cys Leu Asp Phe Gly Trp Glu Asp Leu Ala
            180                 185                 190

tct aca att aat aac atg atg tat ctt gat ata ccg ttt att gct ttt      624
Ser Thr Ile Asn Asn Met Met Tyr Leu Asp Ile Pro Phe Ile Ala Phe
        195                 200                 205

act gca aat aac gat aat tgg gtc aag caa gat gaa gtt atc aca ttg      672
Thr Ala Asn Asn Asp Asn Trp Val Lys Gln Asp Glu Val Ile Thr Leu
    210                 215                 220

tta tca aat att cgt agt aat cga tgc aag ata tat tct ttg tta gga      720
Leu Ser Asn Ile Arg Ser Asn Arg Cys Lys Ile Tyr Ser Leu Leu Gly
225                 230                 235                 240

agt tcg cat gac ttg agt gaa aat tta gtg gtc ctg cgc aat ttt tat      768
Ser Ser His Asp Leu Ser Glu Asn Leu Val Val Leu Arg Asn Phe Tyr
                245                 250                 255

caa tcg gtt acg aaa gcc gct atc gcg atg gat aat gat cat ctg gat      816
Gln Ser Val Thr Lys Ala Ala Ile Ala Met Asp Asn Asp His Leu Asp
            260                 265                 270

att gat gtt gat att act gaa ccg tca ttt gaa cat tta act att gcg      864
Ile Asp Val Asp Ile Thr Glu Pro Ser Phe Glu His Leu Thr Ile Ala
        275                 280                 285

aca gtc aat gaa cgc cga atg aga att gag att gaa aat caa gca att      912
Thr Val Asn Glu Arg Arg Met Arg Ile Glu Ile Glu Asn Gln Ala Ile
    290                 295                 300

tct ctg tct taa                                                      924
Ser Leu Ser
```

305

<210> 6
<211> 307
<212> PRT
<213> P. luminescens

<400> 6

Met Glu Asn Glu Ser Lys Tyr Lys Thr Ile Asp His Val Ile Cys Val
1 5 10 15

Glu Gly Asn Lys Lys Ile His Val Trp Glu Thr Leu Pro Glu Glu Asn
20 25 30

Ser Pro Lys Arg Lys Asn Ala Ile Ile Ile Ala Ser Gly Phe Ala Arg
35 40 45

Arg Met Asp His Phe Ala Gly Leu Ala Glu Tyr Leu Ser Arg Asn Gly
50 55 60

Phe His Val Ile Arg Tyr Asp Ser Leu His His Val Gly Leu Ser Ser
65 70 75 80

Gly Thr Ile Asp Glu Phe Thr Met Ser Ile Gly Lys Gln Ser Leu Leu
85 90 95

Ala Val Val Asp Trp Leu Thr Thr Arg Lys Ile Asn Asn Phe Gly Met
100 105 110

Leu Ala Ser Ser Leu Ser Ala Arg Ile Ala Tyr Ala Ser Leu Ser Glu
115 120 125

Ile Asn Ala Ser Phe Leu Ile Thr Ala Val Gly Val Val Asn Leu Arg
130 135 140

Tyr Ser Leu Glu Arg Ala Leu Gly Phe Asp Tyr Leu Ser Leu Pro Ile
145 150 155 160

Asn Glu Leu Pro Asn Asn Leu Asp Phe Glu Gly His Lys Leu Gly Ala
165 170 175

Glu Val Phe Ala Arg Asp Cys Leu Asp Phe Gly Trp Glu Asp Leu Ala
180 185 190

Ser Thr Ile Asn Asn Met Met Tyr Leu Asp Ile Pro Phe Ile Ala Phe
195 200 205

Thr Ala Asn Asn Asp Asn Trp Val Lys Gln Asp Glu Val Ile Thr Leu

```
    210                 215                 220

Leu Ser Asn Ile Arg Ser Asn Arg Cys Lys Ile Tyr Ser Leu Leu Gly
225                 230                 235                 240

Ser Ser His Asp Leu Ser Glu Asn Leu Val Val Leu Arg Asn Phe Tyr
                245                 250                 255

Gln Ser Val Thr Lys Ala Ala Ile Ala Met Asp Asn Asp His Leu Asp
            260                 265                 270

Ile Asp Val Asp Ile Thr Glu Pro Ser Phe Glu His Leu Thr Ile Ala
        275                 280                 285

Thr Val Asn Glu Arg Arg Met Arg Ile Glu Ile Glu Asn Gln Ala Ile
    290                 295                 300

Ser Leu Ser
305


<210>  7
<211>  924
<212>  DNA
<213>  artificial sequence

<220>
<223>  mutated luxD P. luminescens


<220>
<221>  CDS
<222>  (1)..(924)

<400>  7
atg gaa aat gaa tca aaa tat aaa acc atc gac cac gtt att tgt gtt        48
Met Glu Asn Glu Ser Lys Tyr Lys Thr Ile Asp His Val Ile Cys Val
1               5                   10                  15

gaa gga aat aaa aaa att cat gtt tgg gaa acg ctg cca gaa gaa aac        96
Glu Gly Asn Lys Lys Ile His Val Trp Glu Thr Leu Pro Glu Glu Asn
            20                  25                  30

agc cca aag aga aag aat gcc att att att gcg tct ggt ttt gcc cgc       144
Ser Pro Lys Arg Lys Asn Ala Ile Ile Ile Ala Ser Gly Phe Ala Arg
        35                  40                  45

agg atg gat cat ttt gct ggt ctg gcg gaa tat tta tcg cgg aat gga       192
Arg Met Asp His Phe Ala Gly Leu Ala Glu Tyr Leu Ser Arg Asn Gly
    50                  55                  60

ttt cat gtg atc cgc tat gat tcg ctt cac cac gtt gga ttg agt tca       240
Phe His Val Ile Arg Tyr Asp Ser Leu His His Val Gly Leu Ser Ser
65                  70                  75                  80

ggg aca att gat gaa ttt aca atg tct ata gga aag cag agc ttg tta       288
Gly Thr Ile Asp Glu Phe Thr Met Ser Ile Gly Lys Gln Ser Leu Leu
```

```
                85                  90                  95

gca gtg gtt gat tgg tta act aca cga aaa ata aat aac ttc ggt atg        336
Ala Val Val Asp Trp Leu Thr Thr Arg Lys Ile Asn Asn Phe Gly Met
            100                 105                 110

ttg gct tca agc tta tct gcg cgg ata gct tat gca agc cta tct gaa        384
Leu Ala Ser Ser Leu Ser Ala Arg Ile Ala Tyr Ala Ser Leu Ser Glu
        115                 120                 125

atc aat gct tcg ttt tta atc acc gca gtc ggt gtt gtt aac tta aga        432
Ile Asn Ala Ser Phe Leu Ile Thr Ala Val Gly Val Val Asn Leu Arg
    130                 135                 140

tat tct ctt gaa aga gct tta ggg ttt gat tat ctc agt cta ccc att        480
Tyr Ser Leu Glu Arg Ala Leu Gly Phe Asp Tyr Leu Ser Leu Pro Ile
145                 150                 155                 160

aat gaa ttg ccg aat aat cta gat ttt gaa ggc cat aaa ttg ggt gct        528
Asn Glu Leu Pro Asn Asn Leu Asp Phe Glu Gly His Lys Leu Gly Ala
                165                 170                 175

gaa gtc ttt gcg aga gat tgt ctt gat ttt ggt tgg gaa gat tta gct        576
Glu Val Phe Ala Arg Asp Cys Leu Asp Phe Gly Trp Glu Asp Leu Ala
            180                 185                 190

tct aca att aat aac atg atg tat ctt gat ata ccg ttt att gct ttt        624
Ser Thr Ile Asn Asn Met Met Tyr Leu Asp Ile Pro Phe Ile Ala Phe
        195                 200                 205

act gca aat aac gat aat tgg gtc aag caa gat gaa gtt atc aca ttg        672
Thr Ala Asn Asn Asp Asn Trp Val Lys Gln Asp Glu Val Ile Thr Leu
    210                 215                 220

tta tca aat att cgt agt aat cga tgc aag ata tat tct ttg tta gga        720
Leu Ser Asn Ile Arg Ser Asn Arg Cys Lys Ile Tyr Ser Leu Leu Gly
225                 230                 235                 240

agt tcg cat gac ttg agt gaa aat tta gtg gtc ctg cgc aat ttt tat        768
Ser Ser His Asp Leu Ser Glu Asn Leu Val Val Leu Arg Asn Phe Tyr
                245                 250                 255

caa tcg gtt acg aaa gcc gct atc gcg atg gat aat gat cat ctg gat        816
Gln Ser Val Thr Lys Ala Ala Ile Ala Met Asp Asn Asp His Leu Asp
            260                 265                 270

att gat gtt gat att act gaa ccg tca ttt gaa cat tta act att gcg        864
Ile Asp Val Asp Ile Thr Glu Pro Ser Phe Glu His Leu Thr Ile Ala
        275                 280                 285

aca gtc aat gaa cgc cga atg aga att gag att gaa aat caa gca att        912
Thr Val Asn Glu Arg Arg Met Arg Ile Glu Ile Glu Asn Gln Ala Ile
    290                 295                 300

tct ctg tct taa                                                        924
Ser Leu Ser
305


<210>  8
<211>  307
<212>  PRT
<213>  artificial sequence
```

<220>
<223> Synthetic Construct

<400> 8

Met Glu Asn Glu Ser Lys Tyr Lys Thr Ile Asp His Val Ile Cys Val
1 5 10 15

Glu Gly Asn Lys Lys Ile His Val Trp Glu Thr Leu Pro Glu Glu Asn
20 25 30

Ser Pro Lys Arg Lys Asn Ala Ile Ile Ile Ala Ser Gly Phe Ala Arg
35 40 45

Arg Met Asp His Phe Ala Gly Leu Ala Glu Tyr Leu Ser Arg Asn Gly
50 55 60

Phe His Val Ile Arg Tyr Asp Ser Leu His His Val Gly Leu Ser Ser
65 70 75 80

Gly Thr Ile Asp Glu Phe Thr Met Ser Ile Gly Lys Gln Ser Leu Leu
85 90 95

Ala Val Val Asp Trp Leu Thr Thr Arg Lys Ile Asn Asn Phe Gly Met
100 105 110

Leu Ala Ser Ser Leu Ser Ala Arg Ile Ala Tyr Ala Ser Leu Ser Glu
115 120 125

Ile Asn Ala Ser Phe Leu Ile Thr Ala Val Gly Val Val Asn Leu Arg
130 135 140

Tyr Ser Leu Glu Arg Ala Leu Gly Phe Asp Tyr Leu Ser Leu Pro Ile
145 150 155 160

Asn Glu Leu Pro Asn Asn Leu Asp Phe Glu Gly His Lys Leu Gly Ala
165 170 175

Glu Val Phe Ala Arg Asp Cys Leu Asp Phe Gly Trp Glu Asp Leu Ala
180 185 190

Ser Thr Ile Asn Asn Met Met Tyr Leu Asp Ile Pro Phe Ile Ala Phe
195 200 205

Thr Ala Asn Asn Asp Asn Trp Val Lys Gln Asp Glu Val Ile Thr Leu
210 215 220

Leu Ser Asn Ile Arg Ser Asn Arg Cys Lys Ile Tyr Ser Leu Leu Gly

225 230 235 240

Ser Ser His Asp Leu Ser Glu Asn Leu Val Val Leu Arg Asn Phe Tyr
245 250 255

Gln Ser Val Thr Lys Ala Ala Ile Ala Met Asp Asn Asp His Leu Asp
260 265 270

Ile Asp Val Asp Ile Thr Glu Pro Ser Phe Glu His Leu Thr Ile Ala
275 280 285

Thr Val Asn Glu Arg Arg Met Arg Ile Glu Ile Glu Asn Gln Ala Ile
290 295 300

Ser Leu Ser
305

<210> 9
<211> 1113
<212> DNA
<213> P. luminescens

<220>
<221> CDS
<222> (1)..(1113)

<400> 9
atg act tca tat gtt gat aaa caa gaa att aca gca agc tca gaa att 48
Met Thr Ser Tyr Val Asp Lys Gln Glu Ile Thr Ala Ser Ser Glu Ile
1 5 10 15

gat gat ttg att ttt tcg agc gat cca tta gtg tgg tct tac gac gag 96
Asp Asp Leu Ile Phe Ser Ser Asp Pro Leu Val Trp Ser Tyr Asp Glu
20 25 30

cag gaa aaa atc aga aag aaa ctt gtg ctt gat gca ttt cgt aat cat 144
Gln Glu Lys Ile Arg Lys Lys Leu Val Leu Asp Ala Phe Arg Asn His
35 40 45

tat aaa cat tgt cga gaa tat cgt cac tac tgt cag gca cac aaa gta 192
Tyr Lys His Cys Arg Glu Tyr Arg His Tyr Cys Gln Ala His Lys Val
50 55 60

gat gac aat att acg gaa att gat gac ata cct gta ttc cca aca tcg 240
Asp Asp Asn Ile Thr Glu Ile Asp Asp Ile Pro Val Phe Pro Thr Ser
65 70 75 80

gtt ttt aag ttt act cgc tta tta act tct cag gaa aac gag att gaa 288
Val Phe Lys Phe Thr Arg Leu Leu Thr Ser Gln Glu Asn Glu Ile Glu
85 90 95

agt tgg ttt acc agt agc ggc acg aat ggt tta aaa agt cag gtg gcg 336
Ser Trp Phe Thr Ser Ser Gly Thr Asn Gly Leu Lys Ser Gln Val Ala
100 105 110

cgt gac aga tta agt att gag aga ctc tta ggc tct gtg agt tat ggc 384

```
Arg Asp Arg Leu Ser Ile Glu Arg Leu Leu Gly Ser Val Ser Tyr Gly
        115                 120                 125

atg aaa tat gtt ggt agt tgg ttt gat cat caa ata gaa tta gtc aat      432
Met Lys Tyr Val Gly Ser Trp Phe Asp His Gln Ile Glu Leu Val Asn
    130                 135                 140

ttg gga cca gat aga ttt aat gct cat aat att tgg ttt aaa tat gtt      480
Leu Gly Pro Asp Arg Phe Asn Ala His Asn Ile Trp Phe Lys Tyr Val
145                 150                 155                 160

atg agt ttg gtg gaa ttg tta tat cct acg aca ttt acc gta aca gaa      528
Met Ser Leu Val Glu Leu Leu Tyr Pro Thr Thr Phe Thr Val Thr Glu
                165                 170                 175

gaa cga ata gat ttt gtt aaa aca ttg aat agt ctt gaa cga ata aaa      576
Glu Arg Ile Asp Phe Val Lys Thr Leu Asn Ser Leu Glu Arg Ile Lys
            180                 185                 190

aat caa ggg aaa gat ctt tgt ctt att ggt tcg cca tac ttt att tat      624
Asn Gln Gly Lys Asp Leu Cys Leu Ile Gly Ser Pro Tyr Phe Ile Tyr
        195                 200                 205

tta ctc tgc cat tat atg aaa gat aaa aaa atc tca ttt tct gga gat      672
Leu Leu Cys His Tyr Met Lys Asp Lys Lys Ile Ser Phe Ser Gly Asp
    210                 215                 220

aaa agc ctt tat atc ata acc gga ggc ggc tgg aaa agt tac gaa aaa      720
Lys Ser Leu Tyr Ile Ile Thr Gly Gly Gly Trp Lys Ser Tyr Glu Lys
225                 230                 235                 240

gaa tct ctg aaa cgt gat gat ttc aat cat ctt tta ttt gat act ttc      768
Glu Ser Leu Lys Arg Asp Asp Phe Asn His Leu Leu Phe Asp Thr Phe
                245                 250                 255

aat ctc agt gat att agt cag atc cga gat ata ttt aat caa gtt gaa      816
Asn Leu Ser Asp Ile Ser Gln Ile Arg Asp Ile Phe Asn Gln Val Glu
            260                 265                 270

ctc aac act tgt ttc ttt gag gat gaa atg cag cgt aaa cat gtt ccg      864
Leu Asn Thr Cys Phe Phe Glu Asp Glu Met Gln Arg Lys His Val Pro
        275                 280                 285

ccg tgg gta tat gcg cga gcg ctt gat cct gaa acg ttg aaa cct gta      912
Pro Trp Val Tyr Ala Arg Ala Leu Asp Pro Glu Thr Leu Lys Pro Val
    290                 295                 300

cct gat gga acg ccg ggg ttg atg agt tat atg gat gcg tca gca acc      960
Pro Asp Gly Thr Pro Gly Leu Met Ser Tyr Met Asp Ala Ser Ala Thr
305                 310                 315                 320

agt tat cca gca ttt att gtt acc gat gat gtc ggg ata att agc aga     1008
Ser Tyr Pro Ala Phe Ile Val Thr Asp Asp Val Gly Ile Ile Ser Arg
                325                 330                 335

gaa tat ggt aag tat ccc ggc gtg ctc gtt gaa att tta cgt cgc gtc     1056
Glu Tyr Gly Lys Tyr Pro Gly Val Leu Val Glu Ile Leu Arg Arg Val
            340                 345                 350

aat acg agg acg cag aaa ggg tgt gct tta agc tta acc gaa gcg ttt     1104
Asn Thr Arg Thr Gln Lys Gly Cys Ala Leu Ser Leu Thr Glu Ala Phe
        355                 360                 365
```

gat agt tga 1113
Asp Ser
370

<210> 10
<211> 370
<212> PRT
<213> P. luminescens

<400> 10

```
Met Thr Ser Tyr Val Asp Lys Gln Glu Ile Thr Ala Ser Ser Glu Ile
1               5                   10                  15

Asp Asp Leu Ile Phe Ser Ser Asp Pro Leu Val Trp Ser Tyr Asp Glu
            20                  25                  30

Gln Glu Lys Ile Arg Lys Lys Leu Val Leu Asp Ala Phe Arg Asn His
        35                  40                  45

Tyr Lys His Cys Arg Glu Tyr Arg His Tyr Cys Gln Ala His Lys Val
    50                  55                  60

Asp Asp Asn Ile Thr Glu Ile Asp Asp Ile Pro Val Phe Pro Thr Ser
65                  70                  75                  80

Val Phe Lys Phe Thr Arg Leu Leu Thr Ser Gln Glu Asn Glu Ile Glu
                85                  90                  95

Ser Trp Phe Thr Ser Ser Gly Thr Asn Gly Leu Lys Ser Gln Val Ala
            100                 105                 110

Arg Asp Arg Leu Ser Ile Glu Arg Leu Leu Gly Ser Val Ser Tyr Gly
        115                 120                 125

Met Lys Tyr Val Gly Ser Trp Phe Asp His Gln Ile Glu Leu Val Asn
    130                 135                 140

Leu Gly Pro Asp Arg Phe Asn Ala His Asn Ile Trp Phe Lys Tyr Val
145                 150                 155                 160

Met Ser Leu Val Glu Leu Leu Tyr Pro Thr Thr Phe Thr Val Thr Glu
                165                 170                 175

Glu Arg Ile Asp Phe Val Lys Thr Leu Asn Ser Leu Glu Arg Ile Lys
            180                 185                 190

Asn Gln Gly Lys Asp Leu Cys Leu Ile Gly Ser Pro Tyr Phe Ile Tyr
        195                 200                 205
```

```
Leu Leu Cys His Tyr Met Lys Asp Lys Lys Ile Ser Phe Ser Gly Asp
    210                 215                 220

Lys Ser Leu Tyr Ile Ile Thr Gly Gly Gly Trp Lys Ser Tyr Glu Lys
225                 230                 235                 240

Glu Ser Leu Lys Arg Asp Asp Phe Asn His Leu Leu Phe Asp Thr Phe
                245                 250                 255

Asn Leu Ser Asp Ile Ser Gln Ile Arg Asp Ile Phe Asn Gln Val Glu
            260                 265                 270

Leu Asn Thr Cys Phe Phe Glu Asp Glu Met Gln Arg Lys His Val Pro
        275                 280                 285

Pro Trp Val Tyr Ala Arg Ala Leu Asp Pro Glu Thr Leu Lys Pro Val
    290                 295                 300

Pro Asp Gly Thr Pro Gly Leu Met Ser Tyr Met Asp Ala Ser Ala Thr
305                 310                 315                 320

Ser Tyr Pro Ala Phe Ile Val Thr Asp Asp Val Gly Ile Ile Ser Arg
                325                 330                 335

Glu Tyr Gly Lys Tyr Pro Gly Val Leu Val Glu Ile Leu Arg Arg Val
            340                 345                 350

Asn Thr Arg Thr Gln Lys Gly Cys Ala Leu Ser Leu Thr Glu Ala Phe
        355                 360                 365

Asp Ser
    370


<210>  11
<211>  1083
<212>  DNA
<213>  artificial sequence

<220>
<223>  mutated luxA of P.luminescens


<220>
<221>  CDS
<222>  (1)..(1083)

<400>  11
atg aaa ttt gga aac ttt tta ctt aca tac caa cct ccc caa ttt tct      48
Met Lys Phe Gly Asn Phe Leu Leu Thr Tyr Gln Pro Pro Gln Phe Ser
1               5                   10                  15
```

```
caa aca gag gta atg gaa cgt ttg gtt aaa tta ggt cgc atc tct gag        96
Gln Thr Glu Val Met Glu Arg Leu Val Lys Leu Gly Arg Ile Ser Glu
            20                  25                  30

gag tgt ggt ttt gat acc gta tgg tta ctg gag cat cat ttc acg gag       144
Glu Cys Gly Phe Asp Thr Val Trp Leu Leu Glu His His Phe Thr Glu
        35                  40                  45

ttt ggt cta ctt ggt aac cct tat gtc gct gct gca tat tta ctt ggc       192
Phe Gly Leu Leu Gly Asn Pro Tyr Val Ala Ala Ala Tyr Leu Leu Gly
    50                  55                  60

gcg act aaa aaa ttg aat gta gga acc gcc gct att gtt ctt ccc aca       240
Ala Thr Lys Lys Leu Asn Val Gly Thr Ala Ala Ile Val Leu Pro Thr
65                  70                  75                  80

gcc cat cca gta cgc caa ctt gaa gat gtg aat tta ttg gat caa atg       288
Ala His Pro Val Arg Gln Leu Glu Asp Val Asn Leu Leu Asp Gln Met
                85                  90                  95

tca aaa gga cga ttt cgg ttt ggt att tgc cga ggg ctt tac aac aag       336
Ser Lys Gly Arg Phe Arg Phe Gly Ile Cys Arg Gly Leu Tyr Asn Lys
            100                 105                 110

gac ttt cgc gta ttc ggc gcg gat atg aat aac agt cgc gcc tta gcg       384
Asp Phe Arg Val Phe Gly Ala Asp Met Asn Asn Ser Arg Ala Leu Ala
        115                 120                 125

gaa tgc tgg tac ggg ctg ata aag aat ggc atg aca gag gga tat atg       432
Glu Cys Trp Tyr Gly Leu Ile Lys Asn Gly Met Thr Glu Gly Tyr Met
    130                 135                 140

gaa gct gat aat gaa cat atc aag ttc cat aag gta aaa gta aac ccc       480
Glu Ala Asp Asn Glu His Ile Lys Phe His Lys Val Lys Val Asn Pro
145                 150                 155                 160

gcg gcg tat agc aga ggt ggc gca ccg gtt tat gtg gtg gct gaa tca       528
Ala Ala Tyr Ser Arg Gly Gly Ala Pro Val Tyr Val Val Ala Glu Ser
                165                 170                 175

gct gcg acg act gag tgg gca gct caa ttt ggc cta ccg atg ata tta       576
Ala Ala Thr Thr Glu Trp Ala Ala Gln Phe Gly Leu Pro Met Ile Leu
            180                 185                 190

agt tgg att ata aat act aac gaa aag aaa gca caa ctt gag ctt tat       624
Ser Trp Ile Ile Asn Thr Asn Glu Lys Lys Ala Gln Leu Glu Leu Tyr
        195                 200                 205

aat gag gtg gct caa gaa tat ggg cac gat att cat aat atc gac cat       672
Asn Glu Val Ala Gln Glu Tyr Gly His Asp Ile His Asn Ile Asp His
    210                 215                 220

tgc tta tca tat ata aca tct gta gat cat gac tca att aaa gcg aaa       720
Cys Leu Ser Tyr Ile Thr Ser Val Asp His Asp Ser Ile Lys Ala Lys
225                 230                 235                 240

gag att tgc cgg aaa ttt ctg ggg cat tgg tat gat tct tat gtg aat       768
Glu Ile Cys Arg Lys Phe Leu Gly His Trp Tyr Asp Ser Tyr Val Asn
                245                 250                 255

gct acg act att ttt gat gat tca gac caa aca aga ggt tat gat ttc       816
Ala Thr Thr Ile Phe Asp Asp Ser Asp Gln Thr Arg Gly Tyr Asp Phe
            260                 265                 270
```

```
aat aaa ggg cag tgg cgt gac ttt gta tta aaa gga cat aaa gat act           864
Asn Lys Gly Gln Trp Arg Asp Phe Val Leu Lys Gly His Lys Asp Thr
        275                 280                 285

aat cgc cgt att gat tac agt tac gaa atc aat ccc gtg gga acg ccg           912
Asn Arg Arg Ile Asp Tyr Ser Tyr Glu Ile Asn Pro Val Gly Thr Pro
    290                 295                 300

cag gaa tgt att gac ata att caa aaa gac att gat gct aca gga ata           960
Gln Glu Cys Ile Asp Ile Ile Gln Lys Asp Ile Asp Ala Thr Gly Ile
305                 310                 315                 320

tca aat att tgt tgt gga ttt gaa gct aat gga aca gta gac gaa att          1008
Ser Asn Ile Cys Cys Gly Phe Glu Ala Asn Gly Thr Val Asp Glu Ile
                325                 330                 335

att gct tcc atg aag ctc ttc cag tct gat gtc atg cca ttt ctt aaa          1056
Ile Ala Ser Met Lys Leu Phe Gln Ser Asp Val Met Pro Phe Leu Lys
            340                 345                 350

gaa aaa caa cgt tcg cta tta tat tag                                      1083
Glu Lys Gln Arg Ser Leu Leu Tyr
        355                 360


<210>  12
<211>  360
<212>  PRT
<213>  artificial sequence

<220>
<223>  Synthetic Construct

<400>  12

Met Lys Phe Gly Asn Phe Leu Leu Thr Tyr Gln Pro Pro Gln Phe Ser
1               5                   10                  15

Gln Thr Glu Val Met Glu Arg Leu Val Lys Leu Gly Arg Ile Ser Glu
            20                  25                  30

Glu Cys Gly Phe Asp Thr Val Trp Leu Leu Glu His His Phe Thr Glu
        35                  40                  45

Phe Gly Leu Leu Gly Asn Pro Tyr Val Ala Ala Ala Tyr Leu Leu Gly
    50                  55                  60

Ala Thr Lys Lys Leu Asn Val Gly Thr Ala Ala Ile Val Leu Pro Thr
65                  70                  75                  80

Ala His Pro Val Arg Gln Leu Glu Asp Val Asn Leu Leu Asp Gln Met
                85                  90                  95

Ser Lys Gly Arg Phe Arg Phe Gly Ile Cys Arg Gly Leu Tyr Asn Lys
            100                 105                 110
```

Asp Phe Arg Val Phe Gly Ala Asp Met Asn Asn Ser Arg Ala Leu Ala
115 120 125

Glu Cys Trp Tyr Gly Leu Ile Lys Asn Gly Met Thr Glu Gly Tyr Met
130 135 140

Glu Ala Asp Asn Glu His Ile Lys Phe His Lys Val Lys Val Asn Pro
145 150 155 160

Ala Ala Tyr Ser Arg Gly Gly Ala Pro Val Tyr Val Val Ala Glu Ser
165 170 175

Ala Ala Thr Thr Glu Trp Ala Ala Gln Phe Gly Leu Pro Met Ile Leu
180 185 190

Ser Trp Ile Ile Asn Thr Asn Glu Lys Lys Ala Gln Leu Glu Leu Tyr
195 200 205

Asn Glu Val Ala Gln Glu Tyr Gly His Asp Ile His Asn Ile Asp His
210 215 220

Cys Leu Ser Tyr Ile Thr Ser Val Asp His Asp Ser Ile Lys Ala Lys
225 230 235 240

Glu Ile Cys Arg Lys Phe Leu Gly His Trp Tyr Asp Ser Tyr Val Asn
245 250 255

Ala Thr Thr Ile Phe Asp Asp Ser Asp Gln Thr Arg Gly Tyr Asp Phe
260 265 270

Asn Lys Gly Gln Trp Arg Asp Phe Val Leu Lys Gly His Lys Asp Thr
275 280 285

Asn Arg Arg Ile Asp Tyr Ser Tyr Glu Ile Asn Pro Val Gly Thr Pro
290 295 300

Gln Glu Cys Ile Asp Ile Ile Gln Lys Asp Ile Asp Ala Thr Gly Ile
305 310 315 320

Ser Asn Ile Cys Cys Gly Phe Glu Ala Asn Gly Thr Val Asp Glu Ile
325 330 335

Ile Ala Ser Met Lys Leu Phe Gln Ser Asp Val Met Pro Phe Leu Lys
340 345 350

Glu Lys Gln Arg Ser Leu Leu Tyr
355 360

<210> 13
<211> 984
<212> DNA
<213> artificial sequence

<220>
<223> mutated luxB of P. luminescens

<220>
<221> CDS
<222> (1)..(984)

<400> 13
```
atg aaa ttt gga ttg ttc ttc ctt aac ttc atc aat ccg aca act gtt        48
Met Lys Phe Gly Leu Phe Phe Leu Asn Phe Ile Asn Pro Thr Thr Val
1               5                   10                  15

caa gaa caa agt ata gtt cgc atg cag gaa ata acg gag tat gtt gat        96
Gln Glu Gln Ser Ile Val Arg Met Gln Glu Ile Thr Glu Tyr Val Asp
            20                  25                  30

aag ttg aat ttt gaa cag att tta gtg tat gaa aat cat ttt tca gat       144
Lys Leu Asn Phe Glu Gln Ile Leu Val Tyr Glu Asn His Phe Ser Asp
        35                  40                  45

aat ggt gtt gtc ggc gct cct ctg act gtt tct ggt ttt ctg ctc ggt       192
Asn Gly Val Val Gly Ala Pro Leu Thr Val Ser Gly Phe Leu Leu Gly
    50                  55                  60

tta aca gag aaa att aaa att ggt tca tta aat cac atc att aca act       240
Leu Thr Glu Lys Ile Lys Ile Gly Ser Leu Asn His Ile Ile Thr Thr
65                  70                  75                  80

cat cat cct gtc cgc ata gcg gag gaa gct tgc tta ttg gat cag tta       288
His His Pro Val Arg Ile Ala Glu Glu Ala Cys Leu Leu Asp Gln Leu
                85                  90                  95

agt gaa ggg aga ttt att tta ggg ttt agt gat tgc gaa aaa aaa gat       336
Ser Glu Gly Arg Phe Ile Leu Gly Phe Ser Asp Cys Glu Lys Lys Asp
            100                 105                 110

gaa atg cat ttt ttt aat cgc ccg gct gaa tat caa cag caa cta ttt       384
Glu Met His Phe Phe Asn Arg Pro Ala Glu Tyr Gln Gln Gln Leu Phe
        115                 120                 125

gaa gag tgt tat gaa atc att aac gat gct tta aca aca ggc tat tgt       432
Glu Glu Cys Tyr Glu Ile Ile Asn Asp Ala Leu Thr Thr Gly Tyr Cys
    130                 135                 140

aat cca gat aac gat ttt tat agc ttc cct aaa ata tct gta aat ccc       480
Asn Pro Asp Asn Asp Phe Tyr Ser Phe Pro Lys Ile Ser Val Asn Pro
145                 150                 155                 160

cat gct tat acg cca ggc gga cct cgg aaa tat gta aca gca acc agt       528
His Ala Tyr Thr Pro Gly Gly Pro Arg Lys Tyr Val Thr Ala Thr Ser
                165                 170                 175

cat cat att gtt gag tgg gcg gcc aaa aaa ggt att cct ctc atc ttt       576
His His Ile Val Glu Trp Ala Ala Lys Lys Gly Ile Pro Leu Ile Phe
            180                 185                 190
```

```
aag tgg gat gat tct aat gat gtt aga tat gaa tat gct gaa aga tat         624
Lys Trp Asp Asp Ser Asn Asp Val Arg Tyr Glu Tyr Ala Glu Arg Tyr
        195                 200                 205

aaa gcc gtc gcg gat aaa tat gac gtt gac cta tca gag ata gac cat         672
Lys Ala Val Ala Asp Lys Tyr Asp Val Asp Leu Ser Glu Ile Asp His
    210                 215                 220

cag tta atg ata tta gtt aac tat aac gaa gat agt aat aaa gct aaa         720
Gln Leu Met Ile Leu Val Asn Tyr Asn Glu Asp Ser Asn Lys Ala Lys
225                 230                 235                 240

caa gag acg cgt gca ttt att agt gat tat gtt ctt gaa atg cac cct         768
Gln Glu Thr Arg Ala Phe Ile Ser Asp Tyr Val Leu Glu Met His Pro
                245                 250                 255

aat gaa gat ttc gaa aat aaa ctt gaa gaa ata att gca gaa aac gct         816
Asn Glu Asp Phe Glu Asn Lys Leu Glu Glu Ile Ile Ala Glu Asn Ala
            260                 265                 270

gtc gga aat tat acg gag tgt ata act gcg gct aaa ttg gca att gaa         864
Val Gly Asn Tyr Thr Glu Cys Ile Thr Ala Ala Lys Leu Ala Ile Glu
        275                 280                 285

aag tgt ggt gcg aaa agt gta ttg ctg tcc ttt gaa cca atg aat gat         912
Lys Cys Gly Ala Lys Ser Val Leu Leu Ser Phe Glu Pro Met Asn Asp
    290                 295                 300

ttg atg agc caa aaa aat gta atc aat att gtt gat gat aat att aag         960
Leu Met Ser Gln Lys Asn Val Ile Asn Ile Val Asp Asp Asn Ile Lys
305                 310                 315                 320

aag tac cac atg gaa tat acc taa                                         984
Lys Tyr His Met Glu Tyr Thr
                325


<210>  14
<211>  327
<212>  PRT
<213>  artificial sequence

<220>
<223>  Synthetic Construct

<400>  14

Met Lys Phe Gly Leu Phe Phe Leu Asn Phe Ile Asn Pro Thr Thr Val
1               5                   10                  15


Gln Glu Gln Ser Ile Val Arg Met Gln Glu Ile Thr Glu Tyr Val Asp
            20                  25                  30


Lys Leu Asn Phe Glu Gln Ile Leu Val Tyr Glu Asn His Phe Ser Asp
        35                  40                  45


Asn Gly Val Val Gly Ala Pro Leu Thr Val Ser Gly Phe Leu Leu Gly
    50                  55                  60
```

Leu Thr Glu Lys Ile Lys Ile Gly Ser Leu Asn His Ile Ile Thr Thr
65                  70                  75                  80

His His Pro Val Arg Ile Ala Glu Glu Ala Cys Leu Leu Asp Gln Leu
                85                  90                  95

Ser Glu Gly Arg Phe Ile Leu Gly Phe Ser Asp Cys Glu Lys Lys Asp
            100                 105                 110

Glu Met His Phe Phe Asn Arg Pro Ala Glu Tyr Gln Gln Gln Leu Phe
        115                 120                 125

Glu Glu Cys Tyr Glu Ile Ile Asn Asp Ala Leu Thr Thr Gly Tyr Cys
    130                 135                 140

Asn Pro Asp Asn Asp Phe Tyr Ser Phe Pro Lys Ile Ser Val Asn Pro
145                 150                 155                 160

His Ala Tyr Thr Pro Gly Gly Pro Arg Lys Tyr Val Thr Ala Thr Ser
                165                 170                 175

His His Ile Val Glu Trp Ala Ala Lys Lys Gly Ile Pro Leu Ile Phe
            180                 185                 190

Lys Trp Asp Asp Ser Asn Asp Val Arg Tyr Glu Tyr Ala Glu Arg Tyr
        195                 200                 205

Lys Ala Val Ala Asp Lys Tyr Asp Val Asp Leu Ser Glu Ile Asp His
    210                 215                 220

Gln Leu Met Ile Leu Val Asn Tyr Asn Glu Asp Ser Asn Lys Ala Lys
225                 230                 235                 240

Gln Glu Thr Arg Ala Phe Ile Ser Asp Tyr Val Leu Glu Met His Pro
                245                 250                 255

Asn Glu Asp Phe Glu Asn Lys Leu Glu Glu Ile Ile Ala Glu Asn Ala
            260                 265                 270

Val Gly Asn Tyr Thr Glu Cys Ile Thr Ala Ala Lys Leu Ala Ile Glu
        275                 280                 285

Lys Cys Gly Ala Lys Ser Val Leu Leu Ser Phe Glu Pro Met Asn Asp
    290                 295                 300

Leu Met Ser Gln Lys Asn Val Ile Asn Ile Val Asp Asp Asn Ile Lys
305                 310                 315                 320

```
Lys Tyr His Met Glu Tyr Thr
                325


<210>  15
<211>  774
<212>  DNA
<213>  artificial sequence

<220>
<223>  mutated frp from Vibrio campbellii


<220>
<221>  CDS
<222>  (1)..(774)

<400>  15
atg gtg aag ata cag ccc atc ccc aca act agc cag ggc agc ctt ttt       48
Met Val Lys Ile Gln Pro Ile Pro Thr Thr Ser Gln Gly Ser Leu Phe
1               5                   10                  15

ata atg aat agc acc ata gag aca atc ctg ggc cat aga tcc att agg       96
Ile Met Asn Ser Thr Ile Glu Thr Ile Leu Gly His Arg Ser Ile Arg
            20                  25                  30

aag ttc aca tct gaa cct att gct agt gag cag ctg caa acg att ctt      144
Lys Phe Thr Ser Glu Pro Ile Ala Ser Glu Gln Leu Gln Thr Ile Leu
        35                  40                  45

cag tct ggg ctc gct gct tca agc tca tcc atg ctg cag gtt gtg agt      192
Gln Ser Gly Leu Ala Ala Ser Ser Ser Ser Met Leu Gln Val Val Ser
    50                  55                  60

ata att cgg gtt aca gac acg gaa aag aga aaa ttg ctc gct caa tat      240
Ile Ile Arg Val Thr Asp Thr Glu Lys Arg Lys Leu Leu Ala Gln Tyr
65                  70                  75                  80

gcc ggc aac cag acg tat gtg gaa tcc gct gct gag ttc ctg gtc ttt      288
Ala Gly Asn Gln Thr Tyr Val Glu Ser Ala Ala Glu Phe Leu Val Phe
                85                  90                  95

tgt ata gac tac cag cga cac gct act atc aac ccc gat gtc caa gct      336
Cys Ile Asp Tyr Gln Arg His Ala Thr Ile Asn Pro Asp Val Gln Ala
            100                 105                 110

gac ttt acc gag ctg acc ctg att ggt gca gtg gat tcc ggc ata atg      384
Asp Phe Thr Glu Leu Thr Leu Ile Gly Ala Val Asp Ser Gly Ile Met
        115                 120                 125

gcc cag aat tgc ctc ctg gca gca gaa tca atg ggt ctt ggc gga gtc      432
Ala Gln Asn Cys Leu Leu Ala Ala Glu Ser Met Gly Leu Gly Gly Val
    130                 135                 140

tat atc gga gga ctt cgg aac tca gct gcc caa gtg gat gag ttg ctc      480
Tyr Ile Gly Gly Leu Arg Asn Ser Ala Ala Gln Val Asp Glu Leu Leu
145                 150                 155                 160

gga ctg ccc aag aac aca gct atc ctc ttc gga atg tgc ttg ggg cac      528
Gly Leu Pro Lys Asn Thr Ala Ile Leu Phe Gly Met Cys Leu Gly His
                165                 170                 175
```

```
ccc gat cag agc cct gag aca aag cct aga ctg ccc gct cat gtg atc         576
Pro Asp Gln Ser Pro Glu Thr Lys Pro Arg Leu Pro Ala His Val Ile
            180                 185                 190

gtg cac gag aac caa tat caa gct ctg aac att gac gac gta cag gcg         624
Val His Glu Asn Gln Tyr Gln Ala Leu Asn Ile Asp Asp Val Gln Ala
        195                 200                 205

tat gac aaa aca ttg cag gag tat tat gcc agc aga acc agc aac cag         672
Tyr Asp Lys Thr Leu Gln Glu Tyr Tyr Ala Ser Arg Thr Ser Asn Gln
    210                 215                 220

aag cag agt gtc tgg tcc cag gaa act gca ggc aag ctg gcc gga gaa         720
Lys Gln Ser Val Trp Ser Gln Glu Thr Ala Gly Lys Leu Ala Gly Glu
225                 230                 235                 240

tcc ctc cca cac atc ctg cca tac ctg aac tcc aag ggc ctt gcc aga         768
Ser Leu Pro His Ile Leu Pro Tyr Leu Asn Ser Lys Gly Leu Ala Arg
                245                 250                 255

agg taa                                                                 774
Arg



<210>  16
<211>  257
<212>  PRT
<213>  artificial sequence

<220>
<223>  Synthetic Construct

<400>  16

Met Val Lys Ile Gln Pro Ile Pro Thr Thr Ser Gln Gly Ser Leu Phe
1               5                   10                  15


Ile Met Asn Ser Thr Ile Glu Thr Ile Leu Gly His Arg Ser Ile Arg
            20                  25                  30


Lys Phe Thr Ser Glu Pro Ile Ala Ser Glu Gln Leu Gln Thr Ile Leu
        35                  40                  45


Gln Ser Gly Leu Ala Ala Ser Ser Ser Ser Met Leu Gln Val Val Ser
    50                  55                  60


Ile Ile Arg Val Thr Asp Thr Glu Lys Arg Lys Leu Leu Ala Gln Tyr
65                  70                  75                  80


Ala Gly Asn Gln Thr Tyr Val Glu Ser Ala Ala Glu Phe Leu Val Phe
                85                  90                  95


Cys Ile Asp Tyr Gln Arg His Ala Thr Ile Asn Pro Asp Val Gln Ala
            100                 105                 110
```

Asp Phe Thr Glu Leu Thr Leu Ile Gly Ala Val Asp Ser Gly Ile Met
        115                 120                 125

Ala Gln Asn Cys Leu Leu Ala Ala Glu Ser Met Gly Leu Gly Gly Val
    130                 135                 140

Tyr Ile Gly Gly Leu Arg Asn Ser Ala Ala Gln Val Asp Glu Leu Leu
145                 150                 155                 160

Gly Leu Pro Lys Asn Thr Ala Ile Leu Phe Gly Met Cys Leu Gly His
                165                 170                 175

Pro Asp Gln Ser Pro Glu Thr Lys Pro Arg Leu Pro Ala His Val Ile
            180                 185                 190

Val His Glu Asn Gln Tyr Gln Ala Leu Asn Ile Asp Asp Val Gln Ala
        195                 200                 205

Tyr Asp Lys Thr Leu Gln Glu Tyr Tyr Ala Ser Arg Thr Ser Asn Gln
    210                 215                 220

Lys Gln Ser Val Trp Ser Gln Glu Thr Ala Gly Lys Leu Ala Gly Glu
225                 230                 235                 240

Ser Leu Pro His Ile Leu Pro Tyr Leu Asn Ser Lys Gly Leu Ala Arg
                245                 250                 255

Arg

<210> 17
<211> 6605
<212> DNA
<213> artificial sequence

<220>
<223> operon ilux 2

<400> 17
atgactaaaa aaatttcatt cattattaat ggccaggtag aaatctttcc cgaaagcgat 60

gatttagtgc aatccatcac ttttggtgat aatagtgttt acctgccaat attgaacgac 120

tctcatgtaa aaaccattac tgattgtaat ggaaataacg acttacggtt gcatgacatt 180

gtcaattttc tctatacggt agggcaaaga tggaaaaata atgaataccc aagacgcagg 240

acatacattc gtgacttaaa aaaatatatg ggatattcag aagaaatggc taagctagag 300

gccaattgga tatctatgat attatgttct aaaggcggcc tttatgatgt tgtagaaaat 360

gaacttggtt ctcgccatat catggatgaa tggatacctc agggtgaaag ttatgttcgg 420

gcttttccga aaggtaaatc cgtacatctg ttggcaggta atgttccatt atctgggatc 480
atgtctatat tacgtgcaat tttaactaag aatcagtgta ttataaaaac atcgtcaacc 540
gatcctttta ccgctaatgc attagcttta agttttattg atgtagaccc taatcatccg 600
ataacgcgct ctttatctgt tatatattgg ccccaccaag gtgatatatc actcgcaaaa 660
gaaattatgc gacatgcgga tgttattgtc gcatggggag ggccagatgc gattaattgg 720
gcggtagagc atgcgccatc taatgctgat gtgattaaat ttggtcctaa aaagagcctt 780
tgcattatcg ataatcctgt tgatctgacg tctgcagcga caggtgcggc tcatgatgtt 840
tgtttttacg atcagcgagc ttgcttttct gcccaaaaca tatattatat gggaaatcat 900
tatgaggaat ttaagttggc attgatagaa aaacttaatc tatatgcgca tatattaccg 960
aatgcaaaaa aagattttga tgaaaaggcg gcctattctt tagttcaaaa agagagcttg 1020
tttgccggat taaatgtaga ggcggatatt catcaacgtt ggacgattat tgagtcagat 1080
gcaggtgtgg aatttaatca accacttggc agatgtgtgt accttcatca cgtcgataat 1140
attgagcaaa tattgcctta tgttcaaaaa aataagacgc aaaccatatc tatttttcct 1200
tgggagtcat catttaaata tcgagatgcg ttagcattag aaggtgcgga aaggattgta 1260
gaagcaggaa tgaataacat atttcgagtt ggtggatctc atgacggaat gagaccgttg 1320
caacgattag tgaagtatat ttctcatgaa aggccatcta actatacggc taaggatgtt 1380
gcggttgaaa tagaacagac tcgtttcctg gaagaagaca agttccttgt atttgtccca 1440
taataggtaa aaagtatgga aaaagaatca aaatataaaa ccatcgacca cgttatttgt 1500
gttgaaggaa ataaaaaaat tcatgtttgg gaaacgctgc cagaagaaaa cagcccaaag 1560
agaaagaatg ccattattat tgcgtctggt tttgcccgca ggatggatca ttttgctgga 1620
ctggcggaat atttatcgcg gaatgggttt catgtgatcc gctatgattc gcttcaccac 1680
gttggattga gttcagggac aattgatgaa tttacaatgt ctacaggaaa gcagagcttg 1740
ttagcagtgg ttgattggtt aactacacga aatataagta acttcggtgt gttggcttca 1800
agcttatctg cgcggatagc ttatgcaagc ctatctgaaa tcaatgcttc gtttttaatc 1860
accgcagtcg gtgttgttaa cttaagatat tctcttgaaa gagctttagg gtttgattat 1920
ctcagtcttc ccattaatga attgccgaaa aatctagttt ttgaaggcca taaattgggt 1980
gctgaagtct tcgcgagaga ttgtcttgat tttggttggg aagacttagc ttctacaatt 2040
aataacatga tgtatcttga tataccgttt attgctttta ccgcaaataa cgacaattgg 2100
gtcaagcaag atgaagttat cacattgtta tcaaatattc gaagtaatcg atgtaggata 2160
tattctttgt taggaagttc gcatgacttg agtgaaaatt tagtggccct gcgcaatttt 2220
tatcaatcgg ttacgaaagc cgctatcgcg atggataatg atcatctgga tattaatgtt 2280

gatattactg aaccgtcatt tgaacattta actattgcga cagtcaatga acgccgaatg 2340
agaattgaga ttgtaaatca agcaatttct ctgtcttaaa atctattgag atattctatc 2400
actcaaatag caatataagg actctctgaa ttcatgaaat ttggaaactt tttacttaca 2460
taccaacctc cccaattttc tcaaacagag gtaatggaac gtttggttaa attaggtcgc 2520
atctctgagg agtgtggttt tgataccgta tggttactgg agcatcattt cacggagttt 2580
ggtctacttg gtaaccctta tgtcgctgct gcatatttac ttggcgcgac taaaaaattg 2640
aatgtaggaa ccgccgctat tgttcttccc acagcccatc cagtacgcca acttgaagat 2700
gtgaatttat tggatcaaat gtcaaaagga cgatttcggt ttggtatttg ccgagggctt 2760
tacaacaagg actttcgcgt attcggcgcg gatatgaata acagtcgcgc cttagcggaa 2820
tgctggtacg ggctgataaa gaatggcatg acagagggat atatggaagc tgataatgaa 2880
catatcaagt tccataaggt aaaagtaaac cccgcggcgt atagcagagg tggcgcaccg 2940
gtttatgtgg tggctgaatc agctgcgacg actgagtggg cagctcaatt tggcctaccg 3000
atgatattaa gttggattat aaatactaac gaaaagaaag cacaacttga gctttataat 3060
gaggtggctc aagaatatgg gcacgatatt cataatatcg accattgctt atcatatata 3120
acatctgtag atcatgactc aattaaagcg aaagagattt gccggaaatt tctggggcat 3180
tggtatgatt cttatgtgaa tgctacgact atttttgatg attcagacca aacaagaggt 3240
tatgatttca ataaagggca gtggcgtgac tttgtattaa aaggacataa agatactaat 3300
cgccgtattg attacagtta cgaaatcaat cccgtgggaa cgccgcagga atgtattgac 3360
ataattcaaa aagacattga tgctacagga atatcaaata tttgttgtgg atttgaagct 3420
aatggaacag tagacgaaat tattgcttcc atgaagctct tccagtctga tgtcatgcca 3480
tttcttaaag aaaaacaacg ttcgctatta tattagctaa ggagaaagaa atgaaatttg 3540
gattgttctt ccttaacttc atcaatccga caactgttca agaacaaagt atagttcgca 3600
tgcaggaaat aacggagtat gttgataagt tgaattttga acagatttta gtgtatgaaa 3660
atcatttttc agataatggt gttgtcggcg ctcctctgac tgtttctggt tttctgctcg 3720
gtttaacaga gaaaattaaa attggttcat taaatcacat cattacaact catcatcctg 3780
tccgcatagc ggaggaagct tgcttattgg atcagttaag tgaagggaga tttattttag 3840
ggtttagtga ttgcgaaaaa aaagatgaaa tgcatttttt taatcgcccg gctgaatatc 3900
aacagcaact atttgaagag tgttatgaaa tcattaacga tgctttaaca acaggctatt 3960
gtaatccaga taacgatttt tatagcttcc ctaaaatatc tgtaaatccc catgcttata 4020
cgccaggcgg acctcggaaa tatgtaacag caaccagtca tcatattgtt gagtgggcgg 4080
ccaaaaaagg tattcctctc atctttaagt gggatgattc taatgatgtt agatatgaat 4140
atgctgaaag atataaagcc gtcgcggata aatatgacgt tgacctatca gagatagacc 4200

atcagttaat gatattagtt aactataacg aagatagtaa taaagctaaa caagagacgc 4260
gtgcatttat tagtgattat gttcttgaaa tgcaccctaa tgaagatttc gaaaataaac 4320
ttgaagaaat aattgcagaa aacgctgtcg gaaattatac ggagtgtata actgcggcta 4380
aattggcaat tgaaaagtgt ggtgcgaaaa gtgtattgct gtcctttgaa ccaatgaatg 4440
atttgatgag ccaaaaaaat gtaatcaata ttgttgatga taatattaag aagtaccaca 4500
tggaatatac ctaaccatgg tagatttcga gttgcagcga ggcggcaagt gaacgaatcc 4560
ccaggagcat agataactat gtgactgggg tgagtgaaag cagccaacaa agcagcagct 4620
tgaaagatga agggtataaa agagtatgac agcagtgctg ccatactttc taatattatc 4680
ttgaggagta aaacaggtat gacttcatat gttgataaac aagaaattac agcaagctca 4740
gaaattgatg atttgatttt ttcgagcgat ccattagtgt ggtcttacga cgagcaggaa 4800
aaaatcagaa agaaacttgt gcttgatgca tttcgtaatc attataaaca ttgtcgagaa 4860
tatcgtcact actgtcaggc acacaaagta gatgacaata ttacggaaat tgatgacata 4920
cctgtattcc caacatcggt ttttaagttt actcgcttat taacttctca ggaaaacgag 4980
attgaaagtt ggtttaccag tagcggcacg aatggtttaa aaagtcaggt ggcgcgtgac 5040
agattaagta ttgagagact cttaggctct gtgagttatg gcatgaaata tgttggtagt 5100
tggtttgatc atcaaataga attagtcaat ttgggaccag atagatttaa tgctcataat 5160
atttggttta aatatgttat gagtttggtg gaattgttat atcctacgac atttaccgta 5220
acagaagaac gaatagattt tgttaaaaca ttgaatagtc ttgaacgaat aaaaaatcaa 5280
gggaaagatc tttgtcttat tggttcgcca tactttattt atttactctg ccattatatg 5340
aaagataaaa aaatctcatt ttctggagat aaaagccttt atatcataac cggaggcggc 5400
tggaaaagtt acgaaaaaga atctctgaaa cgtgatgatt tcaatcatct tttatttgat 5460
actttcaatc tcagtgatat tagtcagatc cgagatatat ttaatcaagt tgaactcaac 5520
acttgtttct ttgaggatga aatgcagcgt aaacatgttc cgccgtgggt atatgcgcga 5580
gcgcttgatc ctgaaacgtt gaaacctgta cctgatggaa cgccggggtt gatgagttat 5640
atggatgcgt cagcaaccag ttatccagca tttattgtta ccgatgatgt cgggataatt 5700
agcagagaat atggtaagta tcccggcgtg ctcgttgaaa ttttacgtcg cgtcaatacg 5760
aggacgcaga aagggtgtgc tttaagctta accgaagcgt ttgatagttg agtcgaccta 5820
aggagaaaga aatggtgaag atacagccca tccccacaac tagccagggc agccttttta 5880
taatgaatag caccatagag acaatcctgg gccatagatc cattaggaag ttcacatctg 5940
aacctattgc tagtgagcag ctgcaaacga ttcttcagtc tgggctcgct gcttcaagct 6000
catccatgct gcaggttgtg agtataattc gggttacaga cacggaaaag agaaaattgc 6060

```
tcgctcaata tgccggcaac cagacgtatg tggaatccgc tgctgagttc ctggtctttt      6120

gtatagacta ccagcgacac gctactatca accccgatgt ccaagctgac tttaccgagc      6180

tgaccctgat tggtgcagtg gattccggca taatggccca gaattgcctc ctggcagcag      6240

aatcaatggg tcttggcgga gtctatatcg gaggacttcg gaactcagct gcccaagtgg      6300

atgagttgct cggactgccc aagaacacag ctatcctctt cggaatgtgc ttggggcacc      6360

ccgatcagag ccctgagaca aagcctagac tgcccgctca tgtgatcgtg cacgagaacc      6420

aatatcaagc tctgaacatt gacgacgtac aggcgtatga caaaacattg caggagtatt      6480

atgccagcag aaccagcaac cagaagcaga gtgtctggtc ccaggaaact gcaggcaagc      6540

tggccggaga atccctccca cacatcctgc catacctgaa ctccaagggc cttgccagaa      6600

ggtaa                                                                  6605


<210>  18
<211>  1443
<212>  DNA
<213>  artificial sequence

<220>
<223>  mutated luxC of P. luminescens


<220>
<221>  CDS
<222>  (1)..(1443)

<400>  18
atg act aaa aaa att tca ttc att att aat ggc cag gta gaa atc ttt        48
Met Thr Lys Lys Ile Ser Phe Ile Ile Asn Gly Gln Val Glu Ile Phe
1               5                   10                  15

ccc gaa agc gat gat tta gtg caa tcc atc act ttt ggt gat aat agt        96
Pro Glu Ser Asp Asp Leu Val Gln Ser Ile Thr Phe Gly Asp Asn Ser
            20                  25                  30

gtt tac ctg cca ata ttg aac gac tct cat gta aaa acc att act gat       144
Val Tyr Leu Pro Ile Leu Asn Asp Ser His Val Lys Thr Ile Thr Asp
        35                  40                  45

tgt aat gga aat aac gac tta cgg ttg cat gac att gtc aat ttt ctc       192
Cys Asn Gly Asn Asn Asp Leu Arg Leu His Asp Ile Val Asn Phe Leu
    50                  55                  60

tat acg gta ggg caa aga tgg aaa aat aat gaa tac cca aga cgc agg       240
Tyr Thr Val Gly Gln Arg Trp Lys Asn Asn Glu Tyr Pro Arg Arg Arg
65                  70                  75                  80

aca tac att cgt gac tta aaa aaa tat atg gga tat tca gaa gaa atg       288
Thr Tyr Ile Arg Asp Leu Lys Lys Tyr Met Gly Tyr Ser Glu Glu Met
                85                  90                  95

gct aag cta gag gcc aat tgg ata tct atg ata tta tgt tct aaa ggc       336
Ala Lys Leu Glu Ala Asn Trp Ile Ser Met Ile Leu Cys Ser Lys Gly
            100                 105                 110
```

```
ggc ctt tat gat gtt gta gaa aat gaa ctt ggt tct cgc cat atc atg      384
Gly Leu Tyr Asp Val Val Glu Asn Glu Leu Gly Ser Arg His Ile Met
        115                 120                 125

gat gaa tgg ata cct cag ggt gaa agt tat gtt cgg gct ttt ccg aaa      432
Asp Glu Trp Ile Pro Gln Gly Glu Ser Tyr Val Arg Ala Phe Pro Lys
    130                 135                 140

ggt aaa tcc gta cat ctg ttg gca ggt aat gtt cca tta tct ggg atc      480
Gly Lys Ser Val His Leu Leu Ala Gly Asn Val Pro Leu Ser Gly Ile
145                 150                 155                 160

atg tct ata tta cgt gca att tta act aag aat cag tgt att ata aaa      528
Met Ser Ile Leu Arg Ala Ile Leu Thr Lys Asn Gln Cys Ile Ile Lys
                165                 170                 175

aca tcg tca acc gat cct ttt acc gct aat gca tta gct tta agt ttt      576
Thr Ser Ser Thr Asp Pro Phe Thr Ala Asn Ala Leu Ala Leu Ser Phe
            180                 185                 190

att gat gta gac cct aat cat ccg ata acg cgc tct tta tct gtt ata      624
Ile Asp Val Asp Pro Asn His Pro Ile Thr Arg Ser Leu Ser Val Ile
        195                 200                 205

tat tgg ccc cac caa ggt gat ata tca ctc gca aaa gaa att atg cga      672
Tyr Trp Pro His Gln Gly Asp Ile Ser Leu Ala Lys Glu Ile Met Arg
    210                 215                 220

cat gcg gat gtt att gtc gca tgg gga ggg cca gat gcg att aat tgg      720
His Ala Asp Val Ile Val Ala Trp Gly Gly Pro Asp Ala Ile Asn Trp
225                 230                 235                 240

gcg gta gag cat gcg cca tct aat gct gat gtg att aaa ttt ggt cct      768
Ala Val Glu His Ala Pro Ser Asn Ala Asp Val Ile Lys Phe Gly Pro
                245                 250                 255

aaa aag agc ctt tgc att atc gat aat cct gtt gat ctg acg tct gca      816
Lys Lys Ser Leu Cys Ile Ile Asp Asn Pro Val Asp Leu Thr Ser Ala
            260                 265                 270

gcg aca ggt gcg gct cat gat gtt tgt ttt tac gat cag cga gct tgc      864
Ala Thr Gly Ala Ala His Asp Val Cys Phe Tyr Asp Gln Arg Ala Cys
        275                 280                 285

ttt tct gcc caa aac ata tat tat atg gga aat cat tat gag gaa ttt      912
Phe Ser Ala Gln Asn Ile Tyr Tyr Met Gly Asn His Tyr Glu Glu Phe
    290                 295                 300

aag ttg gca ttg ata gaa aaa ctt aat cta tat gcg cat ata tta ccg      960
Lys Leu Ala Leu Ile Glu Lys Leu Asn Leu Tyr Ala His Ile Leu Pro
305                 310                 315                 320

aat gca aaa aaa gat ttt gat gaa aag gcg gcc tat tct tta gtt caa     1008
Asn Ala Lys Lys Asp Phe Asp Glu Lys Ala Ala Tyr Ser Leu Val Gln
                325                 330                 335

aaa gag agc ttg ttt gcc gga tta aat gta gag gcg gat att cat caa     1056
Lys Glu Ser Leu Phe Ala Gly Leu Asn Val Glu Ala Asp Ile His Gln
            340                 345                 350

cgt tgg acg att att gag tca gat gca ggt gtg gaa ttt aat caa cca     1104
Arg Trp Thr Ile Ile Glu Ser Asp Ala Gly Val Glu Phe Asn Gln Pro
        355                 360                 365
```

```
ctt ggc aga tgt gtg tac ctt cat cac gtc gat aat att gag caa ata        1152
Leu Gly Arg Cys Val Tyr Leu His His Val Asp Asn Ile Glu Gln Ile
    370                 375                 380

ttg cct tat gtt caa aaa aat aag acg caa acc ata tct att ttt cct        1200
Leu Pro Tyr Val Gln Lys Asn Lys Thr Gln Thr Ile Ser Ile Phe Pro
385                 390                 395                 400

tgg gag tca tca ttt aaa tat cga gat gcg tta gca tta gaa ggt gcg        1248
Trp Glu Ser Ser Phe Lys Tyr Arg Asp Ala Leu Ala Leu Glu Gly Ala
                405                 410                 415

gaa agg att gta gaa gca gga atg aat aac ata ttt cga gtt ggt gga        1296
Glu Arg Ile Val Glu Ala Gly Met Asn Asn Ile Phe Arg Val Gly Gly
            420                 425                 430

tct cat gac gga atg aga ccg ttg caa cga tta gtg aag tat att tct        1344
Ser His Asp Gly Met Arg Pro Leu Gln Arg Leu Val Lys Tyr Ile Ser
        435                 440                 445

cat gaa agg cca tct aac tat acg gct aag gat gtt gcg gtt gaa ata        1392
His Glu Arg Pro Ser Asn Tyr Thr Ala Lys Asp Val Ala Val Glu Ile
    450                 455                 460

gaa cag act cgt ttc ctg gaa gaa gac aag ttc ctt gta ttt gtc cca        1440
Glu Gln Thr Arg Phe Leu Glu Glu Asp Lys Phe Leu Val Phe Val Pro
465                 470                 475                 480

taa                                                                    1443


<210>  19
<211>  480
<212>  PRT
<213>  artificial sequence

<220>
<223>  Synthetic Construct

<400>  19

Met Thr Lys Lys Ile Ser Phe Ile Ile Asn Gly Gln Val Glu Ile Phe
1               5                   10                  15


Pro Glu Ser Asp Asp Leu Val Gln Ser Ile Thr Phe Gly Asp Asn Ser
            20                  25                  30


Val Tyr Leu Pro Ile Leu Asn Asp Ser His Val Lys Thr Ile Thr Asp
        35                  40                  45


Cys Asn Gly Asn Asn Asp Leu Arg Leu His Asp Ile Val Asn Phe Leu
    50                  55                  60


Tyr Thr Val Gly Gln Arg Trp Lys Asn Asn Glu Tyr Pro Arg Arg Arg
65                  70                  75                  80


Thr Tyr Ile Arg Asp Leu Lys Lys Tyr Met Gly Tyr Ser Glu Glu Met
```

85 90 95

Ala Lys Leu Glu Ala Asn Trp Ile Ser Met Ile Leu Cys Ser Lys Gly
100 105 110

Gly Leu Tyr Asp Val Val Glu Asn Glu Leu Gly Ser Arg His Ile Met
115 120 125

Asp Glu Trp Ile Pro Gln Gly Glu Ser Tyr Val Arg Ala Phe Pro Lys
130 135 140

Gly Lys Ser Val His Leu Leu Ala Gly Asn Val Pro Leu Ser Gly Ile
145 150 155 160

Met Ser Ile Leu Arg Ala Ile Leu Thr Lys Asn Gln Cys Ile Ile Lys
165 170 175

Thr Ser Ser Thr Asp Pro Phe Thr Ala Asn Ala Leu Ala Leu Ser Phe
180 185 190

Ile Asp Val Asp Pro Asn His Pro Ile Thr Arg Ser Leu Ser Val Ile
195 200 205

Tyr Trp Pro His Gln Gly Asp Ile Ser Leu Ala Lys Glu Ile Met Arg
210 215 220

His Ala Asp Val Ile Val Ala Trp Gly Gly Pro Asp Ala Ile Asn Trp
225 230 235 240

Ala Val Glu His Ala Pro Ser Asn Ala Asp Val Ile Lys Phe Gly Pro
245 250 255

Lys Lys Ser Leu Cys Ile Ile Asp Asn Pro Val Asp Leu Thr Ser Ala
260 265 270

Ala Thr Gly Ala Ala His Asp Val Cys Phe Tyr Asp Gln Arg Ala Cys
275 280 285

Phe Ser Ala Gln Asn Ile Tyr Tyr Met Gly Asn His Tyr Glu Glu Phe
290 295 300

Lys Leu Ala Leu Ile Glu Lys Leu Asn Leu Tyr Ala His Ile Leu Pro
305 310 315 320

Asn Ala Lys Lys Asp Phe Asp Glu Lys Ala Ala Tyr Ser Leu Val Gln
325 330 335

```
Lys Glu Ser Leu Phe Ala Gly Leu Asn Val Glu Ala Asp Ile His Gln
            340                 345                 350

Arg Trp Thr Ile Ile Glu Ser Asp Ala Gly Val Glu Phe Asn Gln Pro
        355                 360                 365

Leu Gly Arg Cys Val Tyr Leu His His Val Asp Asn Ile Glu Gln Ile
    370                 375                 380

Leu Pro Tyr Val Gln Lys Asn Lys Thr Gln Thr Ile Ser Ile Phe Pro
385                 390                 395                 400

Trp Glu Ser Ser Phe Lys Tyr Arg Asp Ala Leu Ala Leu Glu Gly Ala
                405                 410                 415

Glu Arg Ile Val Glu Ala Gly Met Asn Asn Ile Phe Arg Val Gly Gly
            420                 425                 430

Ser His Asp Gly Met Arg Pro Leu Gln Arg Leu Val Lys Tyr Ile Ser
        435                 440                 445

His Glu Arg Pro Ser Asn Tyr Thr Ala Lys Asp Val Ala Val Glu Ile
    450                 455                 460

Glu Gln Thr Arg Phe Leu Glu Glu Asp Lys Phe Leu Val Phe Val Pro
465                 470                 475                 480


<210>  20
<211>  924
<212>  DNA
<213>  artificial sequence

<220>
<223>  mutated luxD of P. luminescens


<220>
<221>  CDS
<222>  (1)..(924)

<400>  20
atg gaa aaa gaa tca aaa tat aaa acc atc gac cac gtt att tgt gtt      48
Met Glu Lys Glu Ser Lys Tyr Lys Thr Ile Asp His Val Ile Cys Val
1               5                   10                  15

gaa gga aat aaa aaa att cat gtt tgg gaa acg ctg cca gaa gaa aac      96
Glu Gly Asn Lys Lys Ile His Val Trp Glu Thr Leu Pro Glu Glu Asn
            20                  25                  30

agc cca aag aga aag aat gcc att att att gcg tct ggt ttt gcc cgc     144
Ser Pro Lys Arg Lys Asn Ala Ile Ile Ile Ala Ser Gly Phe Ala Arg
        35                  40                  45

agg atg gat cat ttt gct gga ctg gcg gaa tat tta tcg cgg aat ggg     192
```

```
Arg Met Asp His Phe Ala Gly Leu Ala Glu Tyr Leu Ser Arg Asn Gly
    50                  55                  60

ttt cat gtg atc cgc tat gat tcg ctt cac cac gtt gga ttg agt tca      240
Phe His Val Ile Arg Tyr Asp Ser Leu His His Val Gly Leu Ser Ser
65                  70                  75                  80

ggg aca att gat gaa ttt aca atg tct aca gga aag cag agc ttg tta      288
Gly Thr Ile Asp Glu Phe Thr Met Ser Thr Gly Lys Gln Ser Leu Leu
                85                  90                  95

gca gtg gtt gat tgg tta act aca cga aat ata agt aac ttc ggt gtg      336
Ala Val Val Asp Trp Leu Thr Thr Arg Asn Ile Ser Asn Phe Gly Val
            100                 105                 110

ttg gct tca agc tta tct gcg cgg ata gct tat gca agc cta tct gaa      384
Leu Ala Ser Ser Leu Ser Ala Arg Ile Ala Tyr Ala Ser Leu Ser Glu
        115                 120                 125

atc aat gct tcg ttt tta atc acc gca gtc ggt gtt gtt aac tta aga      432
Ile Asn Ala Ser Phe Leu Ile Thr Ala Val Gly Val Val Asn Leu Arg
    130                 135                 140

tat tct ctt gaa aga gct tta ggg ttt gat tat ctc agt ctt ccc att      480
Tyr Ser Leu Glu Arg Ala Leu Gly Phe Asp Tyr Leu Ser Leu Pro Ile
145                 150                 155                 160

aat gaa ttg ccg aaa aat cta gtt ttt gaa ggc cat aaa ttg ggt gct      528
Asn Glu Leu Pro Lys Asn Leu Val Phe Glu Gly His Lys Leu Gly Ala
                165                 170                 175

gaa gtc ttc gcg aga gat tgt ctt gat ttt ggt tgg gaa gac tta gct      576
Glu Val Phe Ala Arg Asp Cys Leu Asp Phe Gly Trp Glu Asp Leu Ala
            180                 185                 190

tct aca att aat aac atg atg tat ctt gat ata ccg ttt att gct ttt      624
Ser Thr Ile Asn Asn Met Met Tyr Leu Asp Ile Pro Phe Ile Ala Phe
        195                 200                 205

acc gca aat aac gac aat tgg gtc aag caa gat gaa gtt atc aca ttg      672
Thr Ala Asn Asn Asp Asn Trp Val Lys Gln Asp Glu Val Ile Thr Leu
    210                 215                 220

tta tca aat att cga agt aat cga tgt agg ata tat tct ttg tta gga      720
Leu Ser Asn Ile Arg Ser Asn Arg Cys Arg Ile Tyr Ser Leu Leu Gly
225                 230                 235                 240

agt tcg cat gac ttg agt gaa aat tta gtg gcc ctg cgc aat ttt tat      768
Ser Ser His Asp Leu Ser Glu Asn Leu Val Ala Leu Arg Asn Phe Tyr
                245                 250                 255

caa tcg gtt acg aaa gcc gct atc gcg atg gat aat gat cat ctg gat      816
Gln Ser Val Thr Lys Ala Ala Ile Ala Met Asp Asn Asp His Leu Asp
            260                 265                 270

att aat gtt gat att act gaa ccg tca ttt gaa cat tta act att gcg      864
Ile Asn Val Asp Ile Thr Glu Pro Ser Phe Glu His Leu Thr Ile Ala
        275                 280                 285

aca gtc aat gaa cgc cga atg aga att gag att gta aat caa gca att      912
Thr Val Asn Glu Arg Arg Met Arg Ile Glu Ile Val Asn Gln Ala Ile
    290                 295                 300
```

```
tct ctg tct taa                                                      924
Ser Leu Ser
305


<210>  21
<211>  307
<212>  PRT
<213>  artificial sequence

<220>
<223>  Synthetic Construct

<400>  21

Met Glu Lys Glu Ser Lys Tyr Lys Thr Ile Asp His Val Ile Cys Val
1               5                   10                  15


Glu Gly Asn Lys Lys Ile His Val Trp Glu Thr Leu Pro Glu Glu Asn
            20                  25                  30


Ser Pro Lys Arg Lys Asn Ala Ile Ile Ile Ala Ser Gly Phe Ala Arg
        35                  40                  45


Arg Met Asp His Phe Ala Gly Leu Ala Glu Tyr Leu Ser Arg Asn Gly
    50                  55                  60


Phe His Val Ile Arg Tyr Asp Ser Leu His His Val Gly Leu Ser Ser
65                  70                  75                  80


Gly Thr Ile Asp Glu Phe Thr Met Ser Thr Gly Lys Gln Ser Leu Leu
                85                  90                  95


Ala Val Val Asp Trp Leu Thr Thr Arg Asn Ile Ser Asn Phe Gly Val
            100                 105                 110


Leu Ala Ser Ser Leu Ser Ala Arg Ile Ala Tyr Ala Ser Leu Ser Glu
        115                 120                 125


Ile Asn Ala Ser Phe Leu Ile Thr Ala Val Gly Val Val Asn Leu Arg
    130                 135                 140


Tyr Ser Leu Glu Arg Ala Leu Gly Phe Asp Tyr Leu Ser Leu Pro Ile
145                 150                 155                 160


Asn Glu Leu Pro Lys Asn Leu Val Phe Glu Gly His Lys Leu Gly Ala
                165                 170                 175


Glu Val Phe Ala Arg Asp Cys Leu Asp Phe Gly Trp Glu Asp Leu Ala
            180                 185                 190
```

Ser Thr Ile Asn Asn Met Met Tyr Leu Asp Ile Pro Phe Ile Ala Phe
195 200 205

Thr Ala Asn Asn Asp Asn Trp Val Lys Gln Asp Glu Val Ile Thr Leu
210 215 220

Leu Ser Asn Ile Arg Ser Asn Arg Cys Arg Ile Tyr Ser Leu Leu Gly
225 230 235 240

Ser Ser His Asp Leu Ser Glu Asn Leu Val Ala Leu Arg Asn Phe Tyr
245 250 255

Gln Ser Val Thr Lys Ala Ala Ile Ala Met Asp Asn Asp His Leu Asp
260 265 270

Ile Asn Val Asp Ile Thr Glu Pro Ser Phe Glu His Leu Thr Ile Ala
275 280 285

Thr Val Asn Glu Arg Arg Met Arg Ile Glu Ile Val Asn Gln Ala Ile
290 295 300

Ser Leu Ser
305

<210> 22
<211> 1083
<212> DNA
<213> P. luminescens

<220>
<221> CDS
<222> (1)..(1083)

<400> 22
atg aaa ttt gga aac ttt ttg ctt aca tac caa cct ccc caa ttt tct 48
Met Lys Phe Gly Asn Phe Leu Leu Thr Tyr Gln Pro Pro Gln Phe Ser
1 5 10 15

caa aca gag gta atg aaa cgt ttg gtt aaa tta ggt cgc atc tct gag 96
Gln Thr Glu Val Met Lys Arg Leu Val Lys Leu Gly Arg Ile Ser Glu
20 25 30

gag tgt ggt ttt gat acc gta tgg tta ctg gag cat cat ttc acg gag 144
Glu Cys Gly Phe Asp Thr Val Trp Leu Leu Glu His His Phe Thr Glu
35 40 45

ttt ggt ttg ctt ggt aac cct tat gtc gct gct gca tat tta ctt ggc 192
Phe Gly Leu Leu Gly Asn Pro Tyr Val Ala Ala Ala Tyr Leu Leu Gly
50 55 60

gcg act aaa aaa ttg aat gta gga act gcc gct att gtt ctt ccc aca 240
Ala Thr Lys Lys Leu Asn Val Gly Thr Ala Ala Ile Val Leu Pro Thr
65 70 75 80

```
gcc cat cca gta cgc caa ctt gaa gat gtg aat tta ttg gat caa atg      288
Ala His Pro Val Arg Gln Leu Glu Asp Val Asn Leu Leu Asp Gln Met
                85                  90                  95

tca aaa gga cga ttt cgg ttt ggt att tgc cga ggg ctt tac aac aag      336
Ser Lys Gly Arg Phe Arg Phe Gly Ile Cys Arg Gly Leu Tyr Asn Lys
            100                 105                 110

gac ttt cgc gta ttc ggc aca gat atg aat aac agt cgc gcc tta gcg      384
Asp Phe Arg Val Phe Gly Thr Asp Met Asn Asn Ser Arg Ala Leu Ala
        115                 120                 125

gaa tgc tgg tac ggg ctg ata aag aat ggc atg aca gag gga tat atg      432
Glu Cys Trp Tyr Gly Leu Ile Lys Asn Gly Met Thr Glu Gly Tyr Met
    130                 135                 140

gaa gct gat aat gaa cat atc aag ttc cat aag gta aaa gta aac ccc      480
Glu Ala Asp Asn Glu His Ile Lys Phe His Lys Val Lys Val Asn Pro
145                 150                 155                 160

gcg gcg tat agc aga ggt ggc gca ccg gtt tat gtg gtg gct gaa tca      528
Ala Ala Tyr Ser Arg Gly Gly Ala Pro Val Tyr Val Val Ala Glu Ser
                165                 170                 175

gct tcg acg act gag tgg gct gct caa ttt ggc cta ccg atg ata tta      576
Ala Ser Thr Thr Glu Trp Ala Ala Gln Phe Gly Leu Pro Met Ile Leu
            180                 185                 190

agt tgg att ata aat act aac gaa aag aaa gca caa ctt gag ctt tat      624
Ser Trp Ile Ile Asn Thr Asn Glu Lys Lys Ala Gln Leu Glu Leu Tyr
        195                 200                 205

aat gaa gtg gct caa gaa tat ggg cac gat att cat aat atc gac cat      672
Asn Glu Val Ala Gln Glu Tyr Gly His Asp Ile His Asn Ile Asp His
    210                 215                 220

tgc tta tca tat ata aca tct gta gat cat gac tca att aaa gcg aaa      720
Cys Leu Ser Tyr Ile Thr Ser Val Asp His Asp Ser Ile Lys Ala Lys
225                 230                 235                 240

gag att tgc cgg aaa ttt ctg ggg cat tgg tat gat tct tat gtg aat      768
Glu Ile Cys Arg Lys Phe Leu Gly His Trp Tyr Asp Ser Tyr Val Asn
                245                 250                 255

gct acg act att ttt gat gat tca gac caa aca aga ggt tat gat ttc      816
Ala Thr Thr Ile Phe Asp Asp Ser Asp Gln Thr Arg Gly Tyr Asp Phe
            260                 265                 270

aat aaa ggg cag tgg cgt gac ttt gta tta aaa gga cat aaa gat act      864
Asn Lys Gly Gln Trp Arg Asp Phe Val Leu Lys Gly His Lys Asp Thr
        275                 280                 285

aat cgc cgt att gat tac agt tac gaa atc aat ccc gtg gga acg ccg      912
Asn Arg Arg Ile Asp Tyr Ser Tyr Glu Ile Asn Pro Val Gly Thr Pro
    290                 295                 300

cag gaa tgt att gac ata att caa aaa gac att gat gct aca gga ata      960
Gln Glu Cys Ile Asp Ile Ile Gln Lys Asp Ile Asp Ala Thr Gly Ile
305                 310                 315                 320

tca aat att tgt tgt gga ttt gaa gct aat gga aca gta gac gaa att     1008
Ser Asn Ile Cys Cys Gly Phe Glu Ala Asn Gly Thr Val Asp Glu Ile
                325                 330                 335
```

```
att gct tcc atg aag ctc ttc cag tct gat gtc atg cca ttt ctt aaa        1056
Ile Ala Ser Met Lys Leu Phe Gln Ser Asp Val Met Pro Phe Leu Lys
            340                 345                 350

gaa aaa caa cgt tcg cta tta tat tag                                    1083
Glu Lys Gln Arg Ser Leu Leu Tyr
        355                 360


<210>  23
<211>  360
<212>  PRT
<213>  P. luminescens

<400>  23

Met Lys Phe Gly Asn Phe Leu Leu Thr Tyr Gln Pro Pro Gln Phe Ser
1               5                   10                  15


Gln Thr Glu Val Met Lys Arg Leu Val Lys Leu Gly Arg Ile Ser Glu
            20                  25                  30


Glu Cys Gly Phe Asp Thr Val Trp Leu Leu Glu His His Phe Thr Glu
        35                  40                  45


Phe Gly Leu Leu Gly Asn Pro Tyr Val Ala Ala Ala Tyr Leu Leu Gly
    50                  55                  60


Ala Thr Lys Lys Leu Asn Val Gly Thr Ala Ala Ile Val Leu Pro Thr
65                  70                  75                  80


Ala His Pro Val Arg Gln Leu Glu Asp Val Asn Leu Leu Asp Gln Met
                85                  90                  95


Ser Lys Gly Arg Phe Arg Phe Gly Ile Cys Arg Gly Leu Tyr Asn Lys
            100                 105                 110


Asp Phe Arg Val Phe Gly Thr Asp Met Asn Asn Ser Arg Ala Leu Ala
        115                 120                 125


Glu Cys Trp Tyr Gly Leu Ile Lys Asn Gly Met Thr Glu Gly Tyr Met
    130                 135                 140


Glu Ala Asp Asn Glu His Ile Lys Phe His Lys Val Lys Val Asn Pro
145                 150                 155                 160


Ala Ala Tyr Ser Arg Gly Gly Ala Pro Val Tyr Val Val Ala Glu Ser
                165                 170                 175


Ala Ser Thr Thr Glu Trp Ala Ala Gln Phe Gly Leu Pro Met Ile Leu
            180                 185                 190
```

```
Ser Trp Ile Ile Asn Thr Asn Glu Lys Lys Ala Gln Leu Glu Leu Tyr
        195                 200                 205

Asn Glu Val Ala Gln Glu Tyr Gly His Asp Ile His Asn Ile Asp His
    210                 215                 220

Cys Leu Ser Tyr Ile Thr Ser Val Asp His Asp Ser Ile Lys Ala Lys
225                 230                 235                 240

Glu Ile Cys Arg Lys Phe Leu Gly His Trp Tyr Asp Ser Tyr Val Asn
                245                 250                 255

Ala Thr Thr Ile Phe Asp Asp Ser Asp Gln Thr Arg Gly Tyr Asp Phe
            260                 265                 270

Asn Lys Gly Gln Trp Arg Asp Phe Val Leu Lys Gly His Lys Asp Thr
        275                 280                 285

Asn Arg Arg Ile Asp Tyr Ser Tyr Glu Ile Asn Pro Val Gly Thr Pro
    290                 295                 300

Gln Glu Cys Ile Asp Ile Ile Gln Lys Asp Ile Asp Ala Thr Gly Ile
305                 310                 315                 320

Ser Asn Ile Cys Cys Gly Phe Glu Ala Asn Gly Thr Val Asp Glu Ile
                325                 330                 335

Ile Ala Ser Met Lys Leu Phe Gln Ser Asp Val Met Pro Phe Leu Lys
            340                 345                 350

Glu Lys Gln Arg Ser Leu Leu Tyr
        355                 360


<210>  24
<211>  984
<212>  DNA
<213>  P. luminescens


<220>
<221>  CDS
<222>  (1)..(984)

<400>  24
atg aaa ttt gga ttg ttc ttc ctt aac ttc atc aat tca aca act gtt         48
Met Lys Phe Gly Leu Phe Phe Leu Asn Phe Ile Asn Ser Thr Thr Val
1               5                   10                  15

caa gaa caa agt ata gtt cgc atg cag gaa ata acg gag tat gtt gat         96
Gln Glu Gln Ser Ile Val Arg Met Gln Glu Ile Thr Glu Tyr Val Asp
```

```
            20                  25                  30

aag ttg aat ttt gaa cag att tta gtg tat gaa aat cat ttt tca gat          144
Lys Leu Asn Phe Glu Gln Ile Leu Val Tyr Glu Asn His Phe Ser Asp
        35                  40                  45

aat ggt gtt gtc ggc gct cct ctg act gtt tct ggt ttt ctg ctc ggt          192
Asn Gly Val Val Gly Ala Pro Leu Thr Val Ser Gly Phe Leu Leu Gly
    50                  55                  60

tta aca gag aaa att aaa att ggt tca tta aat cac atc att aca act          240
Leu Thr Glu Lys Ile Lys Ile Gly Ser Leu Asn His Ile Ile Thr Thr
65                  70                  75                  80

cat cat cct gtc cgc ata gcg gag gaa gct tgc tta ttg gat cag tta          288
His His Pro Val Arg Ile Ala Glu Glu Ala Cys Leu Leu Asp Gln Leu
                85                  90                  95

agt gaa ggg aga ttt att tta ggg ttt agt gat tgc gaa aaa aaa gat          336
Ser Glu Gly Arg Phe Ile Leu Gly Phe Ser Asp Cys Glu Lys Lys Asp
            100                 105                 110

gaa atg cat ttt ttt aat cgc ccg gtt gaa tat caa cag caa cta ttt          384
Glu Met His Phe Phe Asn Arg Pro Val Glu Tyr Gln Gln Gln Leu Phe
        115                 120                 125

gaa gag tgt tat gaa atc att aac gat gct tta aca aca ggc tat tgt          432
Glu Glu Cys Tyr Glu Ile Ile Asn Asp Ala Leu Thr Thr Gly Tyr Cys
    130                 135                 140

aat cca gat aac gat ttt tat agc ttc cct aaa ata tct gta aat ccc          480
Asn Pro Asp Asn Asp Phe Tyr Ser Phe Pro Lys Ile Ser Val Asn Pro
145                 150                 155                 160

cat gct tat acg cca ggc gga cct cgg aaa tat gta aca gca acc agt          528
His Ala Tyr Thr Pro Gly Gly Pro Arg Lys Tyr Val Thr Ala Thr Ser
                165                 170                 175

cat cat att gtt gag tgg gcg gcc aaa aaa ggt att cct ctc atc ttt          576
His His Ile Val Glu Trp Ala Ala Lys Lys Gly Ile Pro Leu Ile Phe
            180                 185                 190

aag tgg gat gat tct aat gat gtt aga tat gaa tat gct gaa aga tat          624
Lys Trp Asp Asp Ser Asn Asp Val Arg Tyr Glu Tyr Ala Glu Arg Tyr
        195                 200                 205

aaa gcc gtt gcg gat aaa tat gac gtt gac cta tca gag ata gac cat          672
Lys Ala Val Ala Asp Lys Tyr Asp Val Asp Leu Ser Glu Ile Asp His
    210                 215                 220

cag tta atg ata tta gtt aac tat aac gaa gat agt aat aaa gct aaa          720
Gln Leu Met Ile Leu Val Asn Tyr Asn Glu Asp Ser Asn Lys Ala Lys
225                 230                 235                 240

caa gag acg cgt gca ttt att agt gat tat gtt ctt gaa atg cac cct          768
Gln Glu Thr Arg Ala Phe Ile Ser Asp Tyr Val Leu Glu Met His Pro
                245                 250                 255

aat gaa aat ttc gaa aat aaa ctt gaa gaa ata att gca gaa aac gct          816
Asn Glu Asn Phe Glu Asn Lys Leu Glu Glu Ile Ile Ala Glu Asn Ala
            260                 265                 270

gtc gga aat tat acg gag tgt ata act gcg gct aag ttg gca att gaa          864
```

```
Val Gly Asn Tyr Thr Glu Cys Ile Thr Ala Ala Lys Leu Ala Ile Glu
        275                 280                 285

aag tgt ggt gcg aaa agt gta ttg ctg tcc ttt gaa cca atg aat gat        912
Lys Cys Gly Ala Lys Ser Val Leu Leu Ser Phe Glu Pro Met Asn Asp
    290                 295                 300

ttg atg agc caa aaa aat gta atc aat att gtt gat gat aat att aag        960
Leu Met Ser Gln Lys Asn Val Ile Asn Ile Val Asp Asp Asn Ile Lys
305                 310                 315                 320

aag tac cac atg gaa tat acc taa                                        984
Lys Tyr His Met Glu Tyr Thr
                325


<210>  25
<211>  327
<212>  PRT
<213>  P. luminescens

<400>  25

Met Lys Phe Gly Leu Phe Phe Leu Asn Phe Ile Asn Ser Thr Thr Val
1               5                   10                  15

Gln Glu Gln Ser Ile Val Arg Met Gln Glu Ile Thr Glu Tyr Val Asp
            20                  25                  30

Lys Leu Asn Phe Glu Gln Ile Leu Val Tyr Glu Asn His Phe Ser Asp
        35                  40                  45

Asn Gly Val Val Gly Ala Pro Leu Thr Val Ser Gly Phe Leu Leu Gly
    50                  55                  60

Leu Thr Glu Lys Ile Lys Ile Gly Ser Leu Asn His Ile Ile Thr Thr
65                  70                  75                  80

His His Pro Val Arg Ile Ala Glu Glu Ala Cys Leu Leu Asp Gln Leu
                85                  90                  95

Ser Glu Gly Arg Phe Ile Leu Gly Phe Ser Asp Cys Glu Lys Lys Asp
            100                 105                 110

Glu Met His Phe Phe Asn Arg Pro Val Glu Tyr Gln Gln Gln Leu Phe
        115                 120                 125

Glu Glu Cys Tyr Glu Ile Ile Asn Asp Ala Leu Thr Thr Gly Tyr Cys
    130                 135                 140

Asn Pro Asp Asn Asp Phe Tyr Ser Phe Pro Lys Ile Ser Val Asn Pro
145                 150                 155                 160
```

His Ala Tyr Thr Pro Gly Gly Pro Arg Lys Tyr Val Thr Ala Thr Ser
165 170 175

His His Ile Val Glu Trp Ala Ala Lys Lys Gly Ile Pro Leu Ile Phe
180 185 190

Lys Trp Asp Asp Ser Asn Asp Val Arg Tyr Glu Tyr Ala Glu Arg Tyr
195 200 205

Lys Ala Val Ala Asp Lys Tyr Asp Val Asp Leu Ser Glu Ile Asp His
210 215 220

Gln Leu Met Ile Leu Val Asn Tyr Asn Glu Asp Ser Asn Lys Ala Lys
225 230 235 240

Gln Glu Thr Arg Ala Phe Ile Ser Asp Tyr Val Leu Glu Met His Pro
245 250 255

Asn Glu Asn Phe Glu Asn Lys Leu Glu Glu Ile Ile Ala Glu Asn Ala
260 265 270

Val Gly Asn Tyr Thr Glu Cys Ile Thr Ala Ala Lys Leu Ala Ile Glu
275 280 285

Lys Cys Gly Ala Lys Ser Val Leu Leu Ser Phe Glu Pro Met Asn Asp
290 295 300

Leu Met Ser Gln Lys Asn Val Ile Asn Ile Val Asp Asp Asn Ile Lys
305 310 315 320

Lys Tyr His Met Glu Tyr Thr
325

<210> 26
<211> 774
<212> DNA
<213> Vibrio campbellii

<220>
<221> CDS
<222> (1)..(774)

<400> 26
atg gtg aag ata cag ccc atc ccc aca act agc cag ggc agc ctt ttt 48
Met Val Lys Ile Gln Pro Ile Pro Thr Thr Ser Gln Gly Ser Leu Phe
1 5 10 15

ata atg aat agc acc ata gag aca atc ctg ggc cat aga tcc att agg 96
Ile Met Asn Ser Thr Ile Glu Thr Ile Leu Gly His Arg Ser Ile Arg
20 25 30

```
aag ttc aca tct gaa cct att gct agt gag cag ctg caa acg att ctt        144
Lys Phe Thr Ser Glu Pro Ile Ala Ser Glu Gln Leu Gln Thr Ile Leu
        35                  40                  45

cag tct ggg ctc gct gct tca agc tca tcc atg ctg cag gtt gtg agt        192
Gln Ser Gly Leu Ala Ala Ser Ser Ser Ser Met Leu Gln Val Val Ser
    50                  55                  60

ata att cgg gtt aca gac acg gaa aag aga aaa ttg ctc gct caa tat        240
Ile Ile Arg Val Thr Asp Thr Glu Lys Arg Lys Leu Leu Ala Gln Tyr
65                  70                  75                  80

gcc ggc aac cag acg tat gtg gaa tcc gct gct gag ttc ctg gtc ttt        288
Ala Gly Asn Gln Thr Tyr Val Glu Ser Ala Ala Glu Phe Leu Val Phe
                85                  90                  95

tgt ata gac tac cag cga cac gct act atc aac ccc gat gtc caa gct        336
Cys Ile Asp Tyr Gln Arg His Ala Thr Ile Asn Pro Asp Val Gln Ala
            100                 105                 110

gac ttt acc gag ctg acc ctg att ggt gca gtg gat tcc ggc ata atg        384
Asp Phe Thr Glu Leu Thr Leu Ile Gly Ala Val Asp Ser Gly Ile Met
        115                 120                 125

gcc cag aat tgc ctc ctg gca gca gaa tca atg ggt ctt ggc gga gtc        432
Ala Gln Asn Cys Leu Leu Ala Ala Glu Ser Met Gly Leu Gly Gly Val
    130                 135                 140

tat atc gga gga ctt cgg aac tca gct gcc caa gtg gat gag ttg ctc        480
Tyr Ile Gly Gly Leu Arg Asn Ser Ala Ala Gln Val Asp Glu Leu Leu
145                 150                 155                 160

gga ctg ccc aag aac aca gct atc ctc ttc gga atg tgc ttg ggg cac        528
Gly Leu Pro Lys Asn Thr Ala Ile Leu Phe Gly Met Cys Leu Gly His
                165                 170                 175

ccc gat cag agc cct gag aca aag cct aga ctg ccc gct cat gtg atc        576
Pro Asp Gln Ser Pro Glu Thr Lys Pro Arg Leu Pro Ala His Val Ile
            180                 185                 190

gtg cac gag aac caa tat caa gct ctg aac att gac gac gta cag gcg        624
Val His Glu Asn Gln Tyr Gln Ala Leu Asn Ile Asp Asp Val Gln Ala
        195                 200                 205

tat gac aaa aca atg cag gag tat tat gcc agc aga acc agc aac cag        672
Tyr Asp Lys Thr Met Gln Glu Tyr Tyr Ala Ser Arg Thr Ser Asn Gln
    210                 215                 220

aag cag agt gtc tgg tcc cag gaa act gca ggc aag ctg gcc gga gaa        720
Lys Gln Ser Val Trp Ser Gln Glu Thr Ala Gly Lys Leu Ala Gly Glu
225                 230                 235                 240

tcc cgc cca cac atc ctg cca tac ctg aac tcc aag ggc ctt gcc aaa        768
Ser Arg Pro His Ile Leu Pro Tyr Leu Asn Ser Lys Gly Leu Ala Lys
                245                 250                 255

agg taa                                                                774
Arg
```

<210> 27
<211> 257

<212> PRT
<213> Vibrio campbellii

<400> 27

Met Val Lys Ile Gln Pro Ile Pro Thr Thr Ser Gln Gly Ser Leu Phe
1 5 10 15

Ile Met Asn Ser Thr Ile Glu Thr Ile Leu Gly His Arg Ser Ile Arg
20 25 30

Lys Phe Thr Ser Glu Pro Ile Ala Ser Glu Gln Leu Gln Thr Ile Leu
35 40 45

Gln Ser Gly Leu Ala Ala Ser Ser Ser Ser Met Leu Gln Val Val Ser
50 55 60

Ile Ile Arg Val Thr Asp Thr Glu Lys Arg Lys Leu Leu Ala Gln Tyr
65 70 75 80

Ala Gly Asn Gln Thr Tyr Val Glu Ser Ala Ala Glu Phe Leu Val Phe
85 90 95

Cys Ile Asp Tyr Gln Arg His Ala Thr Ile Asn Pro Asp Val Gln Ala
100 105 110

Asp Phe Thr Glu Leu Thr Leu Ile Gly Ala Val Asp Ser Gly Ile Met
115 120 125

Ala Gln Asn Cys Leu Leu Ala Ala Glu Ser Met Gly Leu Gly Gly Val
130 135 140

Tyr Ile Gly Gly Leu Arg Asn Ser Ala Ala Gln Val Asp Glu Leu Leu
145 150 155 160

Gly Leu Pro Lys Asn Thr Ala Ile Leu Phe Gly Met Cys Leu Gly His
165 170 175

Pro Asp Gln Ser Pro Glu Thr Lys Pro Arg Leu Pro Ala His Val Ile
180 185 190

Val His Glu Asn Gln Tyr Gln Ala Leu Asn Ile Asp Asp Val Gln Ala
195 200 205

Tyr Asp Lys Thr Met Gln Glu Tyr Tyr Ala Ser Arg Thr Ser Asn Gln
210 215 220

Lys Gln Ser Val Trp Ser Gln Glu Thr Ala Gly Lys Leu Ala Gly Glu
225 230 235 240

```
Ser Arg Pro His Ile Leu Pro Tyr Leu Asn Ser Lys Gly Leu Ala Lys
                245                 250                 255

Arg
```

```
<210>  28
<211>  20
<212>  DNA
<213>  artificial sequence

<220>
<223>  crispr system target sequence

<400>  28
gcggcctttt cttttcgtgc                                                  20


<210>  29
<211>  80
<212>  DNA
<213>  artificial sequence

<220>
<223>  crispr oligonucleotide

<400>  29
tcatccatca ctaccatcaa atactgagcg gctcaaaaag aaaaggccgc gtctggcacg      60

atgcggacac gatatacggt                                                  80
```

## Description

The present invention relates in a first aspect to a polypeptide or polypeptide complex containing luxC and/or luxD polypeptide, or an isolated nucleic acid molecule, comprising at least nucleic acid sequences encoding the luxC polypeptide with specific mutations compared to the wild type sequence as well as nucleic acid sequences encoding the luxD polypeptide containing amino acid substitutions as well as nucleotide derivatives thereof. Moreover, the polypeptides encoded are disclosed as well as operons, vectors, bacteriophages and viruses containing said nucleic acid sequences. In addition, cells in particular bacterial cells are provided containing the mutated luxC and luxD genes encoding the specific luxC and luxD polypeptides according to the present invention. Moreover, a method of generation of bright autobioluminescent cells is provided as well as kits comprising at least one isolated nucleic acid molecule according to the present invention or operons or vectors or bacteriophages. Finally, the use of molecules according to present inventions, proteins, operons, vectors and bacteriophages or cells in a process of bioluminescence imaging in living cells is provided.

## PRIOR ART

The cellular light emission by the process of bioluminescence can be conveniently used to image living cells. Advantageously, bioluminescence can be imaged easily with low complexity optical systems while background signal is virtually absent due to its independency from external light. Thus, bioluminescence is advantageous compared to fluorescence imaging in samples with high autofluorescence such as living tissue. Moreover, bioluminescence imaging can be used for long term measurements without phototoxicity or photobleaching and also enables the observation of light sensitive cells. The bacterial bioluminescence system enables light production in living cells without an external luciferin. Namely, the enzyme required, the light producing enzyme luciferase and its substrates can be synthesized by the cell itself by simultaneous expression of the following genes: luxA and luxB encoding the subunits of the luciferase, luxC, luxD and luxE encoding the subunits of the fatty acid reductase complex and the frp gene encoding a flavin reductase. Basically, the bioluminescence process includes the steps of reacting of fatty acid reductase complex producing an aldehyde substrate from cellular fatty acids. The second substrate FMNH_2 is generated by the flavin reductase. It is long known that the bacterial bioluminescence system is functional in non-bacterial cell types such as yeast and mammalian cells allowing the use of the bioluminescence system in said cells for imaging purposes.

However, due to its relatively low levels of light emission, many applications of bioluminescence imaging would benefit from an increase of brightness of this system. Moreover, measurements over long time periods require stable systems with improved brightness as well as, preferably, integrating the genes into the bacterial chromosome rather than to express them from a plasmid, which can be lost under non-selective conditions. Namely, a disadvantage of the use of extra-chromosomal plasmids is the requirement of cultivation with selection markers. Typically a need for antibiotic selection avoiding an outbreak of bacterial cells with loss of the respective plasmid is given. Specific application of the bacterial bioluminescence system is the highly sensitive detection of bacteria in different environments, for example, it can be used to image the spreading of bacterial infections within the living host or allows to detect bacterial contaminations in food samples. That is, various applications are possible, however, requiring different properties of the system used. As mentioned, a loss of the system in the cells under investigation should be avoided. Hence, it is desirable to integrate the genes into the chromosomes accordingly. However, in case of chromosomal expression only one copy of the transgene is present per cell which requires improved bioluminescence emission of the system to generate a strong bioluminescence signal. When using extra-chromosomally present plasmids, the copy number thereof is usually much higher, overcoming this general problem of low emission. However, a permanent selection of cells is required usually based on antibiotics. An increase of brightness of luciferase based bioluminescence systems is desired. This is particularly important in applications enabling the reliable detection of even low cell numbers present in the sample.

Recently, an enhanced bacterial bioluminescence has been reported with an *ilux* operon for single cell imaging. Namely, C. Gregor et al., PNAS 115: 962 (2018) describes a system based on several mutations present in the *lux* operon. The authors report on the so called *ilux* system having an increased brightness when expressed in *E. coli.* This system should enable long term imaging of single bacterial cells while simultaneously providing information about cellular viability. The mutations contained in the *ilux* can be found in the luxA, luxB, luxC and frp gene. However, there is still a need to improve the system, in particular, in case of chromosomal expression where only one copy of the transgene is present per cell. In particular, it is desired to allow detection of single *E. coli* cells or other cells in various applications.

## BRIEF DESCRIPTION OF THE PRESENT INVENTION

The present invention aims at providing new mutations present in the lux system which when integrated into the chromosome yield an autonomously bioluminescent bacterial strain with further increased brightness compared to the prior art describing the *ilux* operon mentioned above. In particular, the system according to the present invention expressed in suitable bacterial cells produces sufficient signal for a robust detection of individual single cells and, in addition, allows highly sensitive long term imaging of bacterial propagation without a selection marker.

In a first aspect, the present invention relates to a polypeptide or a polypeptide complex containing at least one of the polypeptides of i) a luxC polypeptide of SEQ ID NO. 2 or SEQ ID NO. 4 containing additionally at least the additional amino acid substitutions of: N45T, I47T, E54D, S77P, D135G, V348A, and K414E; and/or a luxD polypeptide of SEQ ID NO. 6 or SEQ ID NO. 8 containing additionally at least one of the amino acid substitutions of K106N, N108S, M112V, D168V, K234R and E300V or ii) a variant of a polypeptide of i), having at least 90 % identity therewith, whereby the additional amino acid substitutions identified in i) remain unchanged.

In a further aspect, the present invention relates to an isolated nucleic acid molecule comprising,

a) a nucleic acid of SEQ ID NO. 1 or SEQ ID NO. 3 encoding the luxC polypeptide of SEQ ID NO. 2 or SEQ ID NO. 4, respectively, the nucleic acid of SEQ ID NO. 1 or SEQ ID NO. 3 further containing additional mutations encoding at least the following amino acid substitutions in SEQ ID NO. 2 and SEQ ID NO. 4, respectively: N45T, I47T, E54D, S77P, D135G, V348A, and K414E; and a nucleic acid of SEQ ID NO. 5 of SEQ ID NO. 7 encoding the luxD polypeptide of SEQ ID NO. 6 and SEQ ID NO. 8, respectively, containing mutations encoding at least the following amino acids substitutions: K106N, N108S, M112V, D168K, K234R and E300V;

b) a nucleotide sequence having greater than 98 % identity within the sequence of a) whereby the substitutions identified in a) remain present.

In an embodiment of the present invention, the present invention provides an operon comprising at least one of the nucleic acid molecules according to the present invention.

Further, the present invention relates to a vector, bacteriophage or virus comprising at least one of the nucleic acid molecules according to the present invention or an operon according to the present invention.

In addition, a cell, in particular, a bacterial cell, containing at least one of the nucleic acid molecules according to the present invention, an operon according to the present invention or a vector, bacteriophage or virus according to the present invention is provided.

The polypeptide or polypeptide complex according to the present invention contains a polypeptide encoded by a nucleic acid molecule according to the present invention. In an embodiment, the isolated polypeptide or polypeptide complex comprises a sequence of at least one of the peptides of SEQ ID NOs. 19 or 21, optionally, comprising additionally at least one of the polypeptides of SEQ ID NOs. 10, 12, 14, 16, 23, 25, and 27.

Finally, a kit is provided comprising at least the nucleic acid molecule according to the present invention, an operon according to the present invention, a vector, a bacteriophage according to the present invention and, optionally, if required means for introducing one of the above into cells including bacterial cells.

The subject matter of the present invention is particularly useful in a process of bioluminescence imaging in living cells. Preferably, the embodiments of the present invention are for use in detecting and imaging bacterial cells, in particular, single bacterial cells.

Hence, in a further aspect, a method of generation of bright autobioluminescent cells including bacteria is provided whereby said cells have improved lux operon activity and said method comprises the step of introducing the improved lux operon activity into the cells accordingly. In an aspect, the cells are *E. coli* cells.

## BRIEF DESCRIPTION OF THE DRAWINGS

**Figure 1.** Reaction scheme of the bacterial bioluminescence reaction. LuxAB: luciferase, RCHO: fatty aldehyde, RCOOH: fatty acid, FMN: flavin mononucleotide, $FMNH_2$: reduced FMN, $O_2$: molecular oxygen, $H_2O_2$: hydrogen peroxide, FMNOOH: C4a-peroxy-FMN, FMNOOR: FMN-C4a-peroxyhemiacetal, FMNOH: C4a-hydroxy-FMN.

**Figure 2.** Mutagenesis and screening of iluxAB. Plots show the relative bioluminescence signal normalized to iluxAB. (**A**) For mutagenesis of iluxAB, iluxAB was expressed at low levels from the vector pGEX(-) lac p15Aori whereas iluxCDEfrp was simultaneously expressed at high levels from pGEX(-). Repeated rounds of mutagenesis resulted in the brighter variant iluxXAB. (**B**) Relative brightness of iluxAB and iluxXAB expressed from pGEX(-) lac. iluxCDEfrp was expressed from the same vector. (**C**) Relative brightness of iluxAB and iluxXAB expressed from pGEX(-). iluxCDEfrp was expressed from the same vector. Error bars represent SD of 5 different clones.

**Figure 3**. Mutagenesis and screening of iluxCDE. Plots show the relative bioluminescence signal normalized to iluxCDE. (**A**) For mutagenesis of iluxCDE, iluxCDE was expressed at low levels from the vector pGEX(-) lac p15Aori whereas iluxABfrp was simultaneously expressed at high levels from pGEX(-). Repeated rounds of mutagenesis resulted in the brighter variant ilux2CDE. (**B**) Relative brightness of iluxCDE and ilux2CDE expressed from pGEX(-) lac. iluxABfrp was expressed from the same vector. (**C**) Relative brightness of iluxCDE and ilux2CDE expressed from pGEX(-). iluxABfrp was expressed from the same vector. (**D**) Relative brightness of iluxCDE and ilux2CDE expressed from pQE(-). iluxABfrp was expressed from the same vector. Error bars represent SD of 5 different clones.

**Figure 4.** Comparison of luxCDABE wt, ilux and ilux2 in *Pseudomonas fluorescens.* The indicated lux operons were expressed in *P. fluorescens* from the vector pJOE7771.1 at 30 °C. The bioluminescence signal was normalized to the luxCDABE wt operon. Error bars represent SD of 5 different clones.

**Figure 5.** Comparison of bioluminescence emission from the chromosomally expressed operons luxCDABE wt, ilux and ilux2 (left) with ilux expressed from the vector pQE(-) (right) in Top10 cells. The bioluminescence signal was normalized to the luxCDABE wt strain. Error bars represent SD of 5 different clones.

## DETAILED DESCRIPTION OF THE PRESENT INVENTION

The present inventor aims at providing an improved bioluminescence system enabling live production of the luciferin in living cells without an external luciferin. Based on mutagenesis and screening of the relevant proteins involved in the bioluminescence system, the present inventor identified specific mutations present in the luxC and luxD protein producing increased brightness compared to the prior art lux operons. Namely, the mutations, present in the luxC and luxD polypeptide forming part of the fatty acid reductase complex providing the aldehyde substrate for the bioluminescence reaction allow to produce sufficient signal intensity for a robust detection of individual cells over long time. Further, the present inventor demonstrated that the bioluminescence system according to the present invention allows the robust detection of individual cells over a long term even when incorporated the same into the chromosome of the host cell. Thus, long term cultivation and propagation does not require any selection marker which may otherwise influence the cultivation accordingly.

In a first aspect, the present invention relates to a polypeptide or a polypeptide complex containing at least one of the polypeptides of i) a luxC polypeptide of SEQ ID NO. 2 or SEQ ID NO. 4 containing additionally at least the additional amino acid substitutions of: N45T, I47T, E54D, S77P, D135G, V348A, and K414E; and/or a luxD polypeptide of SEQ ID NO. 6 or SEQ ID NO. 8 containing additionally at least one of the amino acid substitutions of K106N, N108S, M112V, D168V, K234R and E300V or ii) a variant of a polypeptide of i), having at least 90 % identity therewith, whereby the additional amino acid substitutions identified in i) remain unchanged.

The present inventor recognized that the luxC polypeptide as defined, the luxD polypeptide as defined alone or in combination allow to generate improved bioluminescence systems. In particular, based on the specific luxC polypeptide and/or luxD polypeptide, it is possible to allow robust detection of individual cells over long time. The bioluminescence system including the polypeptides according to the present invention have superior properties over known bioluminescence systems, e. g. as described by the inventor before. Higher sensitivity of the imaging is possible and, in addition, better time resolution of the measured bioluminescence of cells is possible.

In an embodiment of the polypeptide or the polypeptide complex according to the present invention, the luxC polypeptide contains additionally at least one of the following amino acid substitutions of N27T, L132I, T216I, Y248N, K345N, T445K and/or the luxD polypeptide contains additionally at least one of the following amino acid substitutions of N3K, I90T, N165K, V251A, D274N or a variant of the polypeptide having at least 90 % identity therewith, whereby the additional amino acid substitutions above remain unchanged.

In a further embodiment, the polypeptide or polypeptide complex according to the present invention comprises the luxC polypeptide of SEQ ID NO. 2 containing additionally the substitution of E74N or in the luxC polypeptide of SEQ ID NO. 4 the additional amino acid substitutions of T10N and D74N.

As noted above, the luxC and luxD polypeptides are part of the fatty acid reductase complex which further comprises the luxE polypeptide. Hence, in an embodiment, a polypeptide complex is provided containing the luxC polypeptide according to the present invention, a luxD polypeptide according to the present invention and, additionally, a luxE polypeptide. In an embodiment, the luxE polypeptide is the luxE polypeptide of SEQ ID NO. 10.

Moreover, the present invention provides a polypeptide composition comprising at least one of the polypeptides of luxC and luxD according to the present invention or a polypeptide complex according to the present invention and, additionally, a luxA and luxB polypeptide. The luxA and luxB polypeptide are subunits forming the luciferase enzyme. In an embodiment, the luxA polypeptide is the luxA polypeptide of SEQ ID NO. 12 or SEQ ID NO. 23 and the luxB polypeptide of SEQ ID NO. 14 or SEQ ID NO. 25. The polypeptide composition according to the present invention thus provides the main components of the luciferase based bioluminescence system composed of the luciferase and the fatty acid reductase complex.

In a further embodiment, the present invention relates to a polypeptide composition containing the polypeptide complex as defined herein further comprising a frp polypeptide, like a frp polypeptide of SEQ ID NO. 16 or of SEQ ID NO. 27.

As an alternative of the frp polypeptide, according to the present invention, luxG of bioluminescent bacteria, like *Vibrio harveyi* or *Photobacterium phosphoreum,* fre of *E.coli.* Generally, the frp gene may be derived from *Vibrio campbelli* but also from other bacteria.

The term "identity" as used herein refers to molecules, either nucleic acid molecules or polypeptides, having a sequence identity of a certain amount, namely, of at least 90 %, like at least 92 %, 94 %, 95 %, 96 %, 97 %, 98 %, 99 %, 99.5 % of the nucleic acid sequence or the amino acid sequence it is referred to. Identity refers to the magnitude of identity between two sequences. Identical sequences have the same or similar characteristics. In particular, the same or similar property of the sequences is identified. For example, the identical nucleotide sequence encodes a polypeptide as claimed herein. The identical polypeptide according to the present invention has the same properties in a bioluminescence system based on luciferase as a polypeptide as defined by reference to a sequence and claimed herein. Moreover, identical nucleic acid molecules encode the same polypeptide but may vary in the sequence due to the degeneracy of the genetic code. Further, identity refers to the presence of identical amino acid or nucleic acid molecules in the order as described for the sequence it refers to. That is, in case of at least 90 % identity, 90 % or more of amino acid molecules are identical at the respective positions. Unless otherwise identified, the terms "homology" and "identity" are used herein interchangeably.

The term "substitution" identifies a change of one amino acid to a different amino acid at a specific position following the general convention. Namely, in case of N45T the amino acid N is substituted with the amino acid T at position 45 of the sequence referred to. Further, the substitution may be due to a single mutation in the codon encoding the amino acid or may result from two or three nucleotide changes based on the genetic code accordingly.

In a further aspect, the present invention relates to an isolated nucleic acid molecule comprising

a) a nucleic acid of SEQ ID NO. 1 or SEQ ID NO. 3 encoding the luxC polypeptide of SEQ ID NO. 2 or SEQ ID NO. 4, respectively, the nucleic acid of SEQ ID NO. 1 or SEQ ID NO. 3 further containing additional mutations encoding at least the following amino acid substitutions in SEQ ID NO. 2 and SEQ ID NO. 4, respectively: N45T, I47T, E54D, S77P, D135G, V348A, and K414E;
and a nucleic acid of SEQ ID NO. 5 of SEQ ID NO.7 encoding the luxD polypeptide of SEQ ID NO. 6 and SEQ ID NO. 8, respectively, containing mutations encoding at least the following amino acids substitutions: K106N, N108S, M112V, D168K, K234R and E300V;
b) a nucleotide sequence having greater than 98 % identity within the sequence of a) whereby the substitutions identified in a) remain present.

As used herein, the term "mutation" identifies a change in the nucleic acid sequence of the respective molecules resulting in a change of the genetic code eventually leading to a substitution of an amino acid.

In addition, the isolated nucleic acid molecule may contain nucleic acid changes due to the use of codons of the host cell wherein the isolated nucleic acid molecule is introduced. The skilled person is well aware of the usage of different codons used in eukaryotic und prokaryotic host cells and the necessary changes, if any, required for expression therein accordingly.

The term "comprise" or "contain" according to the present invention allows the presence of other elements. The embodiments of comprising and containing include the embodiment of "consisting of" or "essentially consisting of".

Unless otherwise identified, the terms "a" and "an" as well as "the" used in the context of describing the invention are to be constituted to include both the singular and the plural.

The embodiment of b) of a nucleotide sequence having greater than 98 % identity refers to nucleotide sequences being derivatives of the nucleic acid molecule as defined under a) whereby the sequence has greater than the 98% identity with said sequence of a). This nucleotide sequence of b) includes in any case the mutations and substitutions identified in a) but the 98% identity refers to the remaining part thereof. Of course, the nucleotide sequence being derivative of the nucleic sequence of a) maintains its functionality accordingly. Namely, the derivatives maintain the activity described for the specific protein, here the luxC and luxD protein as part of the fatty acid reductase complex.

In an embodiment, the nucleic acid molecule comprises the nucleic acid sequence mentioned in b) having greater than 99%, 99.5%, 99.6%, 99.7%, 99.8% or 99.9% identity with the respective sequence mentioned. Identity may be given on nucleic acid level or amino acid level. Generally, identity refers to the basis of the amino acid level accordingly.

The isolated nucleic acid molecule according to the present invention may be obtained from biological systems including cell culture or, alternatively, may be synthesised chemically.

In an embodiment of the present invention, the nucleic acid molecule according to the present invention comprises additionally mutations encoding at least one of the following amino acid substitutions of

a) N27T, L132I, T216I, Y248N, K345N, T445K in the luxC polypeptide, and N3K, I90T, N165K, V251A, D274N in the luxD polypeptide; or
b) a nucleotide sequence having greater than 98 % identity with the sequence of a), whereby the substitutions identified in a) and the additional mutations encoding at least the following amino acid substitutions in SEQ ID NO. 2 and SEQ ID NO. 4, respectively: N45T, I47T, E54D, S77P, D135G, V348A, and K414E;
and the mutations encoding at least the following amino acids substitutions in SEQ ID NO.6 and SEQ ID NO. 8, respectively: K106N, N108S, M112V, D168V, K234R and E300V; remain present;

These additional substitutions in combination with the substitutions mentioned above, represents a preferred embodiment of the bioluminescent system.

Moreover, the present invention relates to an isolated nucleic acid molecule according to the present invention wherein based on the wild type sequence of luxC (SEQ ID NO. 1) the additional substitution of amino acid E74N in SEQ ID NO. 2 is present or in case of the iluxC gene (SEQ ID NO. 3), the additional mutations encoding the amino acid substitutions T10N and D74N of SEQ ID NO. 4 are present.

The sequence of SEQ ID NO. 1 refers to the nucleic acid sequence present in wild type *P.luminescens.* The sequence of SEQ ID NO. 3 refers to the iluxC nucleic acid sequence as disclosed in Gregor et al., PNAS 115: 962 (2018) mentioned above. The corresponding polypeptides of SEQ ID NO. 1 and SEQ ID NO. 3 are polypeptides according to SEQ ID NO. 2 corresponding to the wild type sequence from *P.luminescens* and the polypeptide of the iluxC molecule (SEQ ID NO. 4) described by Gregor et al., PNAS 115: 962 (2018) before.

The sequence of SEQ ID NO. 5 refers to the nucleic acid sequence present in wild type *P.luminescens.* The sequence of SEQ ID NO. 7 refers to the iluxD nucleic acid sequence as disclosed in Gregor et al., PNAS 115: 962 (2018) mentioned above. The corresponding polypeptides of SEQ ID NO. 5 and SEQ ID NO. 7 are polypeptides according to SEQ ID NO. 6 corresponding to the wild type sequence from *P.luminescens* and the polypeptide of the iluxD molecule (SEQ ID NO. 8) described by Gregor et al., PNAS 115: 962 (2018) before.

In a further embodiment, the isolated nucleic acid molecule according to the present invention comprises additionally the luxE gene. For example, luxE gene has the sequence of SEQ ID NO. 9 referring to the wild type sequence from

*P.luminescens.*

In an embodiment, the isolated nucleic acid according to the present invention contains the luxC, D and E nucleic acid sequences as mentioned, in particular, the nucleic acid sequence of SEQ ID NO. 18 representing the so called ilux2C sequence encoding the ilux2C polypeptide of SEQ ID NO. 19. In addition, said nucleic acid luxC, D, E, includes the sequence of SEQ ID NO. 20 referring to the ilux2D gene encoding the ilux2D polypeptide of SEQ ID NO. 21. In addition, the luxE gene is the luxE gene of SEQ ID NO. 9 encoding the luxE polypeptide of SEQ ID NO. 10.

If necessary, the isolated nucleic acid molecule according to the present invention as well as the operon defined below, may contain linker sequences. These linker sequences may be sequences necessary for construction of the operon like restriction sites or intergenic sequences present in the wild type operon.

In a further embodiment of the present invention, the isolated nucleic acid molecule according to the present invention comprises the luxA and luxB genes, e.g. luxA and luxB genes from *P.luminescens.* In an embodiment, the luxA and luxB genes are nucleic acid sequences of SEQ ID NO. 11 and 13, respectively, corresponding to the luxA and luxB sequences disclosed in C. Gregor et al., PNAS 115: 962 (2018), alternatively, the luxA and luxB genes correspond to the wild type luxA and luxB genes of SEQ ID NOs. 22 and 24, respectively encoding the polypeptides of SEQ ID NOs. 23 und 25.

In an embodiment, the isolated nucleic acid molecule is a nucleic acid molecule of SEQ ID NO. 17.

In an embodiment of the present invention, the nucleic acid molecules do not contain any Tag structure as known to the skilled person. That is, no Tag sequence like His-Tag or GST is present.

The nucleic acid molecules according to the present invention may be form of an operon. That is, in another aspect of the present invention, an operon comprising at least one of the nucleic acid molecules as defined herein is disclosed. Namely, an operon represents a functioning unit of DNA containing a cluster of genes under the control of a single promoter. The operon may contain further sequences for construction of the genetically engineered operon or intergenic sequences present in the wild type operon. According to the present invention, the operon includes at least the luxC and luxD genes as disclosed herein, e. g. the lux2C and lux2D genes of SEQ ID NOs. 18 and 20, respectively. In an embodiment, the operon is a luxCDE operon or a luxCDABE operon. A specific operon is the nucleic acid molecule of SEQ ID NO. 17 containing additionally a frp gene.

In case of the use of an operon being a luxCDE operon, a second operon may be present in the host cell comprising luxAB.

For example, the operon according to the present invention comprises further the FRP gene in particular, the FRP gene of SEQ ID NO. 15 encoding a polypeptide of SEQ ID NO. 16. Alternatively, the FRP gene corresponds to the wild type FRP gene of Vibrio campbellii according to SEQ ID NO. 26 encoding the polypeptide of SEQ ID NO. 27. Alternatively, the other genes or polypeptides encoding FMN- or flavin reductases may be present, like fre in E.coli.

For example, a first operon is provided being a luxCDE operon with a second operon being a luxAB FRP operon.

In a further aspect, the present invention relates to a vector, bacteriophage or virus according to the present invention. The skilled person is well aware of suitable vectors including suitable nucleic acid based vehicles, like plasmids or RNA virus for chromosomal and extrachromosomal expression.

As used herein, the term "bacteriophage" also known as a "phage" refers to a virus infecting and replicating within bacteria and archaea. Suitable bacteriophages are known from the skilled person, in particular, bacteriophages suitable for infection and replicating in bacteria like *E. coli.*

In addition, the virus may be any type of virus known to the skilled person allowing infection of the target cells including AAV based virus, etc. The vector, bacteriophage or virus according to the present invention comprises an operon according to the present invention or at least one of the nucleic acid molecules according to the present invention. The operon or nucleic acid molecules are introduced into the vector, bacteriophage or virus by known techniques resulting in the suitable vectors including plasmids.

Suitable vectors, like plasmids include expression plasmids for the nucleic acid molecules encoding the lux proteins or FRP. The vector or plasmid may be a plasmid suitable for exochromosomal expression in the host cell. Alternatively, the plasmid allows to introduce the operon or nucleic acid molecules according to the present invention into the chromosome of the host cell accordingly.

In a further aspect, the present invention relates to a cell containing at least one of the isolated nucleic acid molecules according to the present invention or containing the operon according to the present invention or the vector or plasmid according to the present invention or being transfected with the bacteriophage according to the present invention. In an embodiment, the cell is a bacterial cell. Suitable bacterial cells are known to the skilled person, for example, the bacterial cell is *E. coli.*

Moreover, in an embodiment of the present invention, the cell, in particular, a bacterial cell, contain the nucleic acid molecules encoding the luxC and luxD genes and, optionally, the luxE gene in a first operon, while, separately, a second operon containing the luxAB and FRP gene is present. For example, the cells also termed host cells, are cells wherein the operon according to the present invention is introduced by known means. Typically electroporation, lipotransfection or other known methods are applied for introducing the nucleic acid molecules into the host cells.

In an embodiment of the present invention, the cell, e.g. a bacterial cell, as defined herein wherein the luxAB FRP contain the nucleic acid sequence of SEQ ID NO. 11 encoding the amino acid substituents of K22E, T119A and S178A in the luxA polypeptide according to SEQ ID NO. 12, the nucleic acid sequence of SEQ ID NO. 13 encoding the amino acid substitutions of S13P, V121A, N259D in the luxB polypeptide of SEQ ID NO. 14, and the mutations in SEQ ID NO. 15 encoding amino acid substitutions M213L, R242L, K256R of the FRP polypeptide of SEQ ID NO. 16.

In an embodiment, the cell, like the bacterial cell according to the present invention wherein at least one of the nucleic acid molecules or the operon according to the present invention is present, is integrated into the chromosome of the cell, in particular, the bacterial cell.

Depending on the cell, the bioluminescent system formed by polypeptides being part of the bioluminescent system contains the luxC and luxD polypeptides according to the present invention in combination with any of the further components of the same genetic origin as defined herein or, containing at least one of the host polypeptides forming the bioluminescent system accordingly.

Moreover, according to the present invention the isolated polypeptide or a polypeptide complex comprises a polypeptide encoded by a nucleic acid molecule according to the present invention. In an embodiment, the isolated polypeptide or the polypeptide complex according to the present invention comprises a polypeptide of at least one of SEQ ID NO. 19 or 21. In an embodiment, the polypeptide or polypeptide complex according to the present invention includes further at least one of a wild type lux polypeptide of SEQ ID NOs. 2, 6, 10, 23 and 25 and/or at least one of a mutated lux polypeptide of SEQ ID NOs. 4, 8, 12 or 14.

Any of the combinations of the mentioned polypeptides relevant for the bioluminescent system according to the present invention is possible as long as the system includes at least one of the lux2C and lux2D polypeptide according to the present invention.

The polypeptide may include derivatives whereby amino acids may be absent at the N-terminus or C-terminus or may be substituted with different amino acids as long as the functionality of the polypeptide is maintained.

Moreover, the present invention relates to a method of generation of bright autobioluminescent cells having improved lux operon activity. The method comprises the step of introducing at least one isolated nucleic acid molecule according to the present invention, and/or the operon according to the present invention or introducing the vector, like a plasmid or infecting a suitable host cell with the bacteriophage according to the present invention, thus, introducing the nucleic acid encoding the luxC and luxD gene disclosed herein. The skilled person is well aware of suitable method for introducing the components into the cells.

In an embodiment, the method according to the present invention is suitable to generate bright autoluminescent bacterial cells. In a preferred embodiment, the bacterial cells are *E. coli* cells.

In another embodiment, the method of generating bright autoluminescent cells includes the introduction of the nucleic acid molecules, e.g. in form of operons, into the chromosome of the receiving cells or host cells. Integration into the chromosome is well known to the skilled person and suitable methods are available in the art.

Moreover, the present invention relates to a kit comprising at least one of the isolated nucleic acid molecules according to the present invention or an operon according to the present invention or a vector, e.g. in form of plasmids or bacteriophages according to the present invention. For example, the kit comprises the vector with the operon to be introduced or the bacteriophage for infection of the host cells. Optionally, the kit comprises further means for introducing the at least one nucleic acid molecule, the operon and, in particular, the vector or plasmids into the cells. These means include known means for introduction, like liposomes, electroporator or other chemical or physical based methods known to the skilled person.

In a further aspect, the present invention relates to the use of the nucleic acid molecules according to the present invention or the use of the operon according to the present invention or the use of the vector or bacteriophage according to the present invention as well as the use of the cells according to the present invention in a process of bioluminescence imaging of living cells. In an embodiment, the use refers to the bioluminescence imaging in bacterial cells including detecting and imaging bacterial cells. In an embodiment, the use is a use for detecting and imaging single bacterial cells in samples and probes. Depending on the kind of application, detecting and imaging may be in a food sample or may be in tissue *in vitro* or *in vivo* on single cell level. For example in case of pathogenic bacteria detection and imaging of propagation and spreading may be determined. Also imaging of biofilms, e.g. in various application in the medical area including implants may be conducted. Further, imaging and detection of the autoluminescent signals is possible with increased sensitivity, in particular, compared with the lux wildtype system and the ilux system described before. That is, lower numbers of cells can be detected and measured as well as spatial and temporal resolution of the measurement is improved. Also determining viability of cells in various applications is possible, e.g. where cells are used in bioreactors etc. In addition, application in methods of screening and developing active agents like cytostatics or in case of determining and measuring toxic or harmful compounds in environmental technology is possible.

Detection and imaging of the bioluminescence of living cells can be conducted by known means.

The present invention will be described further by way of examples without limiting the same thereto.

**Materials and Methods**

**Calculation of optimal screening conditions.**

To determine the optimal conditions for the screening, a mathematical model was set up based on the reaction scheme in (*Figure* **1**). For better clarity, the following abbreviations are used below: A: aldehyde (RCHO), F: $FMNH_2$, O: $O_2$, L: LuxAB, LA: LuxAB·RCHO, LF: LuxAB·$FMNH_2$, LFO: LuxAB·FMNHOOH, LFOA: LuxAB·FMNHOOR. We first aimed at including also reactions for the production of aldehyde from fatty acids by LuxCDE and of $FMNH_2$ from FMN by Frp in order to express the rate of light emission as a function of the reaction rate constants and concentrations of LuxAB, LuxCDE and Frp. However, since the resulting system was too complex to utilize its analytical solution, we decided to use the concentrations of $FMNH_2$ and aldehyde as variables instead of LuxCDE and Frp activity.

Based on the reaction scheme, the emitted bioluminescence B is proportional to the concentration of the LFOA complex:

$$B = k_9\,[LFOA]$$

Under cellular steady-state conditions, the concentrations of LA, LF, LFO and LFOA remain constant. Therefore, the following rate equations can be deduced from (*Figure* **1**):

$$\frac{d[LA]}{dt} = k_1\,[L]\,[A] - k_2\,[LA] = 0$$

$$\frac{d[LF]}{dt} = k_3\,[L]\,[F] - k_4\,[LF] - k_5\,[O]\,[LF] = 0$$

$$\frac{d[LFO]}{dt} = k_5\,[O]\,[LF] - k_6\,[LFO] - k_7\,[A]\,[LFO] + k_8\,[LFOA] = 0$$

$$\frac{d[LFOA]}{dt} = k_7\,[A]\,[LFO] - k_8\,[LFOA] - k_9\,[LFOA] = 0$$

In addition, the concentrations of all intermediates must equal the total cellular concentration of luciferase $[L]_0$ (for simplicity, other existing states of the luciferase are neglected since their concentration is also constant under steady-state conditions):

$$[L]_0 = [L] + [LA] + [LF] + [LFO] + [LFOA]$$

This set of six equations was solved with a custom-written Matlab (The MathWorks, Inc., Natick, Massachusetts) script. Since the rate constants $k_i$ for the utilized proteins are unknown, they were all set to 1 for a qualitative description of the process, yielding the following equation for the bioluminescence emission:

$$B = \frac{[F]\,[A]\,[O]\,[L]_0}{(2+[A])\cdot(1+[O])\cdot(1+[F]+[A])}$$

From this equation, it is evident that the bioluminescence signal increases less than proportionally with the substrate concentrations [F] and [A], i.e., an increase in [F] or [A] results in a smaller percentage change of B. For optimal sensitivity during the screening, the relative signal change with varying concentrations of the reactants (F, A and $L_0$, respectively) must be maximal. This was calculated as the derivative with respect to the screened reactant and normalized to the signal B, yielding for F:

$$\frac{dB}{d[F]} = \frac{[A]\,[O]\,[L]_0\cdot(1+[A])}{(2+[A])\cdot(1+[O])\cdot(1+[F]+[A])^2}$$

$$\frac{dB/d[F]}{B} = \frac{1+[A]}{[F]\cdot(1+[F]+[A])}$$

and for A:

$$\frac{dB}{d[A]} = \frac{[F]\,[O]\,[L]_0\cdot(2+2[F]-[A]^2)}{(2+[A])^2\cdot(1+[O])\cdot(1+[F]+[A])^2}$$

$$\frac{dB/d[A]}{B} = \frac{2+2[F]-[A]^2}{[A]\cdot(2+[A])\cdot(1+[F]+[A])}$$

These results are plotted in Fig. 1 with [O] and [L]o set to 1.

**Construction of screening vectors.**

The vector pGEX(-) for tagless expression of the Lux proteins was generated as described previously (C. Gregor et al., PNAS 115: 962 (2018)). For the two-plasmid based screening, another expression vector pGEX(-) Kan was generated in which the ampicillin resistance was exchanged against a kanamycin resistance marker in the following way: the kanamycin resistance gene was amplified by PCR using the primers KanR Spel fwd and KanR Ascl rev. The vector backbone was amplified with the primers pGEX(-) Spel rev and pGEX(-) Ascl fwd. Both parts were digested with Spel and Ascl and subsequently ligated.

For low-level expression, the tac promoter in pGEX(-) was exchanged against a lac promoter by PCR of the vector with the primers pGEX(-) +lac BamHI rev and pGEX(-) BamHI fwd and digestion with BamHI, followed by ligation without an insert.

To maintain two plasmids in the same cell during the screening, the ColE1 origin of replication in pGEX(-) was exchanged against the p15A origin of replication (p15Aori). For this purpose, the p15A origin of replication was PCR amplified with the primers p15Aori BglII fwd and p15Aori AvrII rev, whereas the vector backbone was amplified with the primers pGEX(-) BglII rev and pGEX(-) AvrII fwd. Both parts were digested with BglII and AvrII and subsequently ligated.

**Error-prone mutagenesis and screening.**

For mutagenesis of *luxAB*, *iluxAB* was amplified from ilux pGEX(-) described in C. Gregor et al., PNAS 115: 962 (2018) and cloned into the vector pGEX(-) lac p15Aori between the BamHI and NotI restriction sites. The remaining genes *iluxCDEfrp* were cloned into pGEX(-) Kan between the BamHI and NotI restriction sites for high-level expression. Error-prone mutagenesis of *iluxAB* in pGEX(-) lac p15Aori was performed as described previously (C. Gregor et al., PNAS 115: 962 (2018)). For screening, the ligation mixture was transformed into electrocompetent *E. coli* Top10 cells already containing the plasmid *iluxCDEfrp* pGEX(-) Kan. The cells were then spread out on LB agar plates containing ampicillin and kanamycin (both 50 μg/ml) and incubated overnight at 37 °C. Expression was not induced with IPTG. The following day, the plates were imaged with an Amersham Imager 600 RGB (AI 600 RGB, GE Healthcare, Chicago, Illinois) and the clones with the highest bioluminescence signal were selected and used for the next round of mutagenesis. Screening and mutagenesis of *luxCDE* were performed in analogy to *luxAB* using the plasmids *iluxCDE* pGEX(-) lac p15Aori and *iluxABfrp* pGEX(-) Kan.

For mutagenesis of frp, a Top10 *fre* knock-out (freKO) strain was generated in order to reduce $FMNH_2$ production by the endogenous flavin reductase encoded by the *fre* gene. The knock-out strain was constructed by CRISPR using the system described in C.R. Reisch et al., Sci. Rep. 5: 15096 (2015) with the target sequence GCGGCCTTTTCTTTTCGTGC (SEQ ID No, 28) and the oligonucleotide T*C*A*T*CCATCACTACCATCAAATACTGAGC GGCTCAAAAAGAAAAGGCCGCGTCTGGCACGATGCGGACACGATATACGGT (SEQ. ID. NO. 29) for recombineering (* indicates phosphorothioate bonds).

**Comparison of LuxCDE wt, iLuxCDE and iLux2CDE activity with different fatty acids.**

*luxCDE wt, iluxCDE, ilux2CDE* and *iluxABfrp* were expressed in Top10 cells from the vector pGEX(-) Kan. For each construct, four colonies were spread out onto LB agar plates containing 50 μg/ml kanamycin and 20 μM IPTG and incubated overnight at 37 °C. The following day, cells were resuspended in LB medium and the $OD_{600}$ was adjusted 0.5 (*luxCDE wt, iluxCDE, ilux2CDE*) or 1.0 (*iluxABfrp*). 100 μl of the *luxCDE* cell suspension was mixed with 3 μl of fatty acid solution (octanoic, decanoic, dodecanoic, tetradecanoic, hexadecanoic or octadecanoic acid, all dissolved in ethanol at a concentration of 10 mM) and incubated for 5 min at room temperature. Next, 10 μl of the *luxABfrp* cell suspension was added and incubated for 5 min at room temperature before starting the measurement. Bioluminescence intensity was determined with the AI 600 RGB. The backgound signal from a sample without addition of fatty acid was subtracted.

***Lux* expression in *Pseudomonas fluorescens***

*Pseudomonas fluorescens* (DSM 50090) was obtained from DSMZ (Leibniz Institute DSMZ - German Collection of Microorganisms and Cell Cultures GmbH, Braunschweig, Germany) and grown in LB medium at 30 °C. For expression in *P. fluorescens, luxCDABE wt, ilux* and *ilux2* were inserted into the expression vector pJOE7771.1 (J. Hoffmann et al., PLoS One 10: e1033248 (2015)) between the BamHI and SpeI restriction site. Plasmid transformation was performed by electroporation in 2 mm cuvettes at 2.5 kV using a capacitance of 25 μF and a resistance of 200 Ω. Cells were grown at 30 °C on LB agar plates containing 50 μg/ml kanamycin.

**Generation of stably bioluminescent *E. coli* strains.**

For chromosomal insertion, *luxCDABE wt, ilux, ilux2* and EYFP were first cloned into a pGEX(-) vector containing an restriction site in front of the tac promoter. This vector was generated by PCR of pGEX(-) with the primers pGEX(-) tac fwd and pGEX(-) tac rev, followed by digestion of the PCR product with and ligation without an insert. In the next step, *luxCDABE wt, ilux, ilux2* and EYFP were cut from previously generated pGEX(-) plasmids with BamHI and NotI and ligated into the new vector pGEX(-) tac digested with the same enzymes. Finally, the complete inserts including the tac promoter were cut out from the newly generated plasmids using and NotI and ligated into the vector pGRG25 described in G.J. McKenzie et al., BMC Microbiol. 6: 39 (2006) that was digested with the same enzymes.

Insertion of the transgenes from pGRG25 into *E. coli* Top10 was performed as described in G.J. McKenzie et al., BMC Microbiol. 6: 39 (2006). Curing of the pGRG25 plasmid was checked by ampicillin sensitivity. Correct insertion of the transgenes into the chromosome was verified by colony PCR of the final strains using the primers attTn7 fwd and luxC EcoRI rev for *luxCDABE wt, ilux* and *ilux2,* frp SalI fwd and attTn7 rev for *ilux* and *ilux2* and attTn7 fwd and attTn7 rev for EYFP.

**Bioluminescence imaging.**

To compare the brightness of different variants, bacteria were transformed with the indicated plasmids and grown on LB agar plates containing the appropriate antibiotics overnight. The following day, single colonies were spread out onto a new LB agar plate. If not stated otherwise, plates were incubated at 37 °C overnight. The following day, plates were imaged with the AI 600 RGB to quantify the bioluminescence signal.

The number of detectable cells of stable Top10 strains containing *luxCDABE wt, ilux, ilux2* and EYFP on mashed potatoes was compared in 24-well plates. Each well was filled with freshly prepared mashed potatoes (consisting of boiled potatoes and water only) up to a height of around 0.5 cm. Cell grown at 37 °C on LB agar plates without antibiotics or IPTG were resuspended in PBS and 1 μl of different dilutions of the cell suspension was added at the center of each well. Plates were imaged at room temperature with the AI 600 RGB. For the *luxCDABE wt, ilux* and *ilux2* strains, bioluminescence was recorded for 10 min. For the EYFP strain, fluorescence was recorded with an excitation wavelength of 520 nm, a 605BP40 filter and a 1 s exposure time. The number of viable cells was determined by plating different dilutions of the cell suspensions on LB agar plates and counting the number of cfu after incubation overnight at 37 °C.

Imaging of single *E. coli* cells was performed using a custom-built microscope as described previously (C. Gregor et al, PNAS 116: 26491 (2019); C. Gregor et al., PNAS 115: 962 (2018); C. Gregor, Methods Mol. Biol. 2081: 43 (2020)). Briefly, cells grown on LB agar plates were resuspended in LB medium and imaged between a coverslip and LB agar pad. Imaging was performed in a stage top incubation system (Okolab) at 37 °C. A 60× oil immersion objective lens (PlanApo N 60x/1.42, Olympus) and an electron-multiplying charge-coupled device (EMCCD) camera (iXon EMCCD DU-897E-CS0-#BV, Andor) with the camera sensor cooled to -100 °C were used to detect the bioluminescence light. To select and focus the cells, cells were transformed with EYFP pGEX(-) beforehand. EYFP fluorescence was excited with a laser (Calypso 50 mW, Cobolt) at 491 nm. Bright pixels were filtered out using a custom-written Matlab script as described in C. Gregor et al, PNAS 116: 26491 (2019); C. Gregor et al., PNAS 115: 962 (2018); C. Gregor, Methods Mol. Biol. 2081: 43 (2020).

Movies of the stable *ilux2* Top10 strain on food products were taken with the AI 600 RGB. Slices of raw cucumber and potato, raw egg yolk, mashed potatoes (consisting of boiled potatoes and water only) and UHT milk were placed in transparent 6 cm dishes, inoculated with around $5 \cdot 10^6$ cfu in PBS and covered with a lid to prevent dehydration of the samples. One image (exposure time 1 min or 5 s) was recorded per hour for 48 h.

**Results**

**Design of a new screening system.**

Since numerous applications of bioluminescence imaging would benefit from a genetically encodable bioluminescence system with high light output, the brightness obtained with the *luxCDABE* operon from *Photorhabdus luminescens* in *Escherichia coli* has been previously optimized (C. Gregor et al., PNAS 115: 962 (2018)). It was observed that bioluminescence emission in *E. coli* is not only limited by the activity of the luciferase itself, but also by cellular production of the substrates ($FMNH_2$ and fatty aldehyde). Consequently, incorporation of a flavin reductase (*frp*) into the *lux* operon and improving substrate production by several rounds of error-prone mutagenesis of *luxCDE* and *frp* substantially contributed to the increased light emission of the final improved operon *ilux.*

In the previous approach, all *lux* genes were expressed from a single vector pGEX(-) (C. Gregor et al., PNAS 115: 962 (2018)) at relatively high levels during error-prone mutagenesis and screening. By mutagenesis of the individual genes in *ilux* pGEX(-), the authors were not able to identify any additional mutations that further increased the bioluminescence emission. Although this could indicate that the Lux proteins are already optimized, two different explanation for this observation exist: First, supply of ATP and NADPH that are required for recycling of the substrates of the bioluminescence reaction might be limited by the cellular metabolism so that no further increase in light emission can be achieved by modifications of the Lux proteins. However, this explanation can be excluded since bioluminescence was increased by expressing *ilux* at higher levels from the vector pQE(-) instead of pGEX(-) (C. Gregor et al., PNAS 115: 962 (2018)). Second, it is possible that further mutations in *ilux* which enhance the brightness exist, but that their effect is too small to detect them under the screening conditions used, although several such mutations combined might still be able to increase the bioluminescence emission substantially.

To explore this second possibility further, the inventor developed a mathematical model of the bioluminescence reaction in order to determine the screening conditions under which the screening is most sensitive (i.e., even small changes in enzyme activities can be detected). Since the only observable in the screening is the bioluminescence signal rather than the enzyme activities themselves, this implies that the relative increase of the bioluminescence signal with an increase in activity of the iLux proteins should be maximized. For this purpose, the inventor first calculated the rate of light emission B as a function of the substrate concentrations by assuming the reaction scheme in (*Figure* **1**) W.A. Francisco et al., Biochemistry 37: 2596 (1998); R. Tinikul et al., Bioluminescence: Fundamentals and Applications in Biotechnology (Volume 3): 154, 47, Springer (2014)) as explained in the *Materials and Methods* section (*F*: $FMNH_2$, *A*: aldehyde, *O*: molecular oxygen, *L*: luciferase):

$$B = \frac{[F]\,[A]\,[O]\,[L]_0}{(2+[A])\cdot(1+[O])\cdot(1+[F]+[A])} \qquad \textbf{(Equation 1)}$$

The bioluminescence signal first increases with the $FMNH_2$ and aldehyde concentrations and decreases again at high aldehyde levels as a consequence of aldehyde inhibition resulting from reversible formation of a luciferase-aldehyde complex (H.M. Abu-Soud et al., J. Biol. Chem. 268: 7699 (1993)), with the optimal aldehyde concentration depending on the $FMNH_2$ concentration, not shown. As can be seen from (*Equation 1*), bioluminescence increases less than proportionally with the substrate concentrations. Therefore, an improvement in activity of the substrate-producing enzymes is not reflected to the same extent in the bioluminescence signal, and hence even significantly improved variants may not be detectable in the screening. Under ideal screening conditions, the relative increase in enzymatic activity would closely approach the increase in bioluminescence. To determine the optimal conditions for screening, the inventor therefore calculated the relative change of the bioluminescence signal with the concentrations of $FMNH_2$ and aldehyde (see *above).* The relative change in signal is highest when production of the substrate of interest is low and therefore rate-limiting for the overall bioluminescence process, whereas the other substrate is present in excess, data not shown. To implement this in the new screening system, the inventor analyzed the components LuxAB, LuxCDE and Frp separately. The component to be improved by mutagenesis was expressed at low levels from a pGEX(-) vector containing a lac promoter and medium-copy-number p15A origin of replication (ori) whereas the other components were expressed at high levels from a separate plasmid using the stronger tac promoter and high-copy-number ColE1 ori (Table 1).

**Table 1**. Plasmid combinations used for error-prone mutagenesis. pGEX(-) was used as the vector backbone.

| Plasmid for error-prone mutagenesis | | | | Plasmid containing the remaining *lux* genes | | | |
|---|---|---|---|---|---|---|---|
| Insert | Promoter | Origin of replication | Resistance | Insert | Promoter | Origin of replication | Resistance |
| *iluxAB* | lac | p15Aori | ampicillin | *iluxCDEfrp* | tac | ColE1 | kanamycin |
| *iluxCDE* | lac | p15Aori | ampicillin | *iluxABfrp* | tac | ColE1 | kanamycin |
| *ilux frp* | tac | p15Aori | ampicillin | *iluxCDABE* | tac | ColE1 | kanamycin |

**Generation of *ilux2.***

Starting from the previously described *ilux* operon (C. Gregor et al., PNAS 115: 962 (2018)) the three components *iluxAB, iluxCDE* and *ilux frp* were modified by repeated rounds of error-prone mutagenesis in independent screenings. Mutagenesis of *iluxAB* resulted in the new variant *iluxXAB* that contains 18 new mutations (Table 2) and produced fivefold higher bioluminescence signal under screening conditions than *iluxAB (Figure 2A).* However, when expressed at higher levels together with *iluxCDEfrp* from the same vector, *iluxXAB* yielded substantially lower signal than *iluxAB* (*Figure 2B, C*) and inhibited cell growth at high expression levels for unknown reasons. *iluxXAB* was therefore not further investigated.

**Table 2.** Mutations identified during the screening of *luxAB* in addition to the mutations in *ilux.*

| Gene | Mutations |
|---|---|
| *luxA* | L24M, I76V, Q86H, M139L, K283R, N289S, I298T, Y360- |
| *luxB* | T28S, Q95H, D112G, K193R, K238N, Y276F, M306T, V311A, D317A, H323L |

Mutagenesis and screening of *iluxCDE* yielded the improved variant *ilux2CDE* containing 26 new mutations in the *luxC* and *luxD* genes (Table 3).

**Table 3**. Mutations contained in *ilux2* in addition to the mutations in *ilux.*

| Gene | Mutations |
|---|---|
| *ilux2C* | T10N, N27T, N45T, I47T, E54D, D74N, S77P, L132I, D135G, T216I, Y248N, K345N, V348A, K414E, T445K |
| *ilux2D* | N3K, I90T, K106N, N108S, M112V, N165K, D168V, K234R, V251A, D274N, E300V |

Under screening conditions, *ilux2CDE* exhibited a 160-fold higher brightness than *iluxCDE (Figure 3A).* When expressed at increasing levels from the same plasmid as *iluxABfrp (3B-D),* the difference in brightness decreased as expected *(Figure 3B,C)* since LuxCDE activity became less limiting for the bioluminescence process. At very high expression levels, however, the brightness of *ilux2CDE* was decreased in comparison to *ilux (Figure 3D).* Since cell growth was concomitantly reduced, we attribute this to toxic effects resulting from excessive aldehyde production, although additionally aldehyde inhibition of the luciferase cannot be ruled out. The beneficial properties of *ilux2CDE* therefore only prevail if its expression levels are not too high.

Screening of the third component *frp* required special consideration. Whereas Frp activity needed to be low in comparison to LuxAB and LuxCDE for sensitive screening, it simultaneously had to be sufficiently high to be distinguishable from the activity of other cellular flavin reductases. Using the same plasmid combinations for screening as for *luxAB* and *luxCDE*, *ilux frp* expression did not result in bioluminescence distinct from expression of *iluxCDABE* alone. To reduce the background signal resulting from cellular flavin reductases, the inventor therefore engineered an *E. coli* strain in which the flavin reductase gene *fre* was knocked out since Fre has been described to be the major FMN reductase for bioluminescence in *E. coli* (Z.T. Campbell et al., J. Biol. Chem. 284: 8322 (2009)). However, bioluminescence from *iluxCDABE* in this strain was still not different between *ilux frp* pGEX(-) lac p15Aori and the empty vector, indicating that the activity of other cellular flavin reductases was still too high. As a solution to this problem, the inventor increased *ilux frp* expression by exchanging the lac promoter in the screening vector against the stronger tac promoter. The resulting bioluminescence could now clearly be discriminated from the empty vector. Importantly, the brightness was still significantly different from the higher *ilux frp* expression from pGEX(-) Kan so that improvements in Frp could still increase the bioluminescence signal. However, we were not able to identify brighter mutants by error-prone mutagenesis and screening, possibly because *ilux frp* cannot be further improved.

Overall, the brightness of *ilux* could only be improved by the mutations in the fatty acid reductase complex. The inventor named the resulting operon *ilux2* which consists of *ilux2CDE* and *iluxABfrp* arranged in the original order, i.e., *ilux2CD iluxABEfrp.*

To test if iLux2CDE increases the brightness also in bacteria other than *E. coli,* the inventor expressed *luxCDABE wt, ilux* and *ilux2* from the vector pJOE7771.1 (J. Hoffmann et al., PLoS One 10: e1033248 (2015)) in *Pseudomonas fluorescens,* see figure 4. Both *ilux* and *ilux2* exhibited higher brightness than *luxCDABE wt* with bioluminescence emission from *ilux2* being highest, indicating that *ilux2* is also a superior reporter in bacteria other than *E. coli.*

Next, the inventor engineered stable *E. coli* strains stably expressing the different *lux* operons by inserting them into the chromosome. Apart from the *lux* variant, the brightness also strongly depend on its expression levels, and therefore higher bioluminescence can generally be achieved with expression from a plasmid due to the higher copy number per cell. However, stable maintenance of a plasmid usually requires selection with an antibiotic resistance marker, which is not feasible or desirable for many applications such as long-term imaging of bacterial infections in living animals and plants, and hence chromosomal expression is preferable. The inventor therefore used the pGRG25 vector (G.J. McKenzie et al., BMC Microbiol. 6: 39 (2006)) for site-specific chromosomal insertion of the *lux* operons at the *attTn7* site in *E. coli* Top10 cells (*Figure 5*)*.* In the chromosomally labeled strains, bioluminescence from *ilux* was only ~65% higher than from *luxCDABE wt.* One reason for this may be that $FMNH_2$ production by other cellular enzymes is comparatively high at the lower chromosomal expression levels, and therefore its supply is less rate-limiting for the bioluminescence reaction. The brightness of the *ilux2* strain was ~12-fold higher than that of the *luxCDABE wt* strain (~7-fold higher than *ilux).* Compared to the highest levels of light emission that we obtained so far with *ilux* expressed from the vector pQE(-), the *ilux2* strain exhibits 12% of its brightness and should therefore enable highly sensitive and quantifiable detection of *E. coli* cells during long-term imaging without selection pressure.

**Bioluminescence imaging of *ilux2.***

The inventor next applied the *ilux2* strain for bioluminescence imaging in different samples. First, the cell number required for detection in macroscopic samples using a commercial imaging system was determined. For this purpose, the inventor applied different dilutions of the stably bioluminescent *E. coli* strains onto mashed potatoes and imaged them with an Amersham Imager (AI) 600 RGB. Addition of 1 μl of cell suspension resulted in spreading of the cells on the surface of the sample within an area of ~500 μm in diameter. At the resulting cell density, around $1 \cdot 10^4$ and $7 \cdot 10^3$ cells were required to reliably detect the *luxCDABE wt* and *ilux* strains, respectively, whereas only $1 \cdot 10^3$ cells were required for the *ilux2* strain. In contrast, a strain chromosomally labeled with EYFP could not be detected by fluorescence even at the highest cell number of $10^6$ cells used. While bioluminescence imaging is limited by the low number of emitted photons, fluorescence imaging of samples with high autofluorescence such as food products or living tissues mostly suffers from the high fluorescence background. Consequently, discrimination of the fluorescence signal of the EYFP strain was impossible despite the higher brightness of the image, demonstrating the superiority of bioluminescence imaging in these samples.

Imaging with the AI 600 RGB was performed at room temperature instead of 37 °C where bioluminescence levels are 2-3 times higher (C. Gregor et al., PNAS 115: 962 (2018)). In addition, a relatively large distance between sample and objective lens was used to record a large field of view. Therefore, even lower cell numbers should be detectable under different imaging conditions. To test if even single cells can be detected, we imaged the *luxCDABE wt, ilux* and *ilux2* strains with a custom-built microscope as described in C. Gregor et al, PNAS 116: 26491 (2019); C. Gregor et al., PNAS 115: 962 (2018); C. Gregor, Methods Mol. Biol. 2081: 43 (2020). Only the *ilux2* strain could be imaged with a high signal-to-noise ratio, demonstrating the usefulness of this strain also for single-cell applications.

Finally, the inventor applied the *ilux2* strain for long-term imaging of growth of *E. coli* cells in different food samples, data not shown. Since only viable cells emit bioluminescence, both growth and spreading of the cells in the sample can be monitored over time. Cells grown on slices of raw cucumber and potato reached maximum bioluminescence after around 5 and 8 hours, respectively. Interestingly, the signal was not homogeneously distributed over the inoculated region, but especially on potato numerous bright spots appeared that changed their position over time. It is speculated that these bright spots emerge in regions of elevated supply with nutrient such as starch which might originate from breakdown of the potato cells. On mashed potatoes and egg yolk, the bioluminescence signal in the inoculated region was more uniform. The signal strongly increased over time and reached its maximum after more than 24 h, indicating a high nutrient availability that supports long-term growth at high cell numbers. Since movement of the cells on the semi-solid surfaces was very limited, the signal finally started to decay at the center of the inoculated region where nutrients were depleted first whereas at the edges high bioluminescence was sustained for longer times.

**Discussion**

By optimization of the screening conditions, the inventor demonstrate that light emission from the bacterial bioluminescence system can be improved under certain expression conditions compared to the previously engineered *ilux* system (C. Gregor et al., PNAS 115: 962 (2018)). This was achieved by component-wise mutagenesis of the involved proteins and screening under rate-limiting reaction conditions so that smaller improvements became detectable, which added up to a significant enhancement in light emission after multiple screening steps. It should be noted that no absolute value for the improvement in brightness can be specified since it strongly depends on the expression levels of all involved proteins. In addition, the overall process of bioluminescence is affected by the cellular availability of the substrates (e.g., fatty acids), resulting in a complex relationship between *lux* expression and bioluminescence emission. Moreover, toxic effects due to accumulation of the reaction products such as aldehydes can lead to adverse effects and reduce cell growth at elevated expression levels, which in turn decreases light emission. Therefore, bioluminescence from the newly generated *ilux2* operon is only increased at moderate expression levels. Nevertheless, the described screening system enables the generation of improved protein variants that would not be accessible otherwise. We therefore believe that the presented screening strategy will also be useful for the optimization of other processes involving multiple enzymes.

The enhanced light emission of the exemplified *ilux2* system results from 26 mutations in the fatty acid reductase complex (15 mutations in *iluxC,* 11 mutations in *iluxD*). The results demonstrate that the increased light output is at least in part due to the improved utilization of fatty acids other than tetradecanoic acid for which the fatty acid reductase complex has the highest activity (S. Ulitzur et al., PNAS 75: 266 (1978); D. Riendeau et al., J. Biol. Chem. 254: 7488 (1979)). A broadened substrate spectrum of iLux2CDE would be in line with the high specificity of the wild-type fatty acid reductase complex for tetradecanoic acid since fatty acids that are potentially more abundant in the cell could also be used for the process of bioluminescence. The luciferase itself discriminates less between different chain lengths of its aldehyde substrate ((J.W. Hastings et al., J. Biol. Chem. 238: 3100 (1963); J.W. Hastings et al., Biochemistry 8: 4681 (1969)) so that elevated production of aldehydes other than tetradecanoic aldehyde is expected to increase light emission. Besides the saturated fatty acids that were investigated in this study, also other substrates may be used with altered specificity in the cell, such as unsaturated fatty acids or acyl-ACP and acyl-CoA esters.

The increased brightness of *ilux2* is particularly useful for the generation of stably autobioluminescent bacteria by chromosomal integration of the operon. These stable strains could for instance be used to image the spreading of bacterial infections in living animals and plants with enhanced sensitivity. Since bioluminescence emission requires metabolic activity, only live bacteria emit light. Therefore, bioluminescence imaging does not only yield information about the localization of the bacteria within the host, but also about their viability so that processes such as bacterial elimination by drugs or the immune system can be observed. In addition, *ilux2*-labeled strains can be used for *in vitro* applications, e.g. for drug screening to develop new antibacterial agents or to study antibiotic resistances. Another important application is the visualization of pathogenic bacteria on food samples to contribute to food safety. For example, bacterial growth can be studied under different storage conditions over long periods of time and different methods for preserving foodstuffs can be tested. It is expected that *ilux2* will be a valuable reporter for highly sensitive bacterial imaging in samples that are not accessible by fluorescence measurements.

## Claims

1. A polypeptide or a polypeptide complex containing at least one of the polypeptides of i) a luxC polypeptide of SEQ ID NO. 2 or SEQ ID NO. 4 containing additionally at least the additional amino acid substitutions of: N45T, I47T, E54D, S77P, D135G, V348A, and K414E; and/or a luxD polypeptide of SEQ ID NO. 6 or SEQ ID NO. 8 containing additionally at least one of the amino acid substitutions of K106N, N108S, M112V, D168V, K234R and E300V or ii) a variant of a polypeptide of i), having at least 90 % identity therewith, whereby the additional amino acid substitutions identified in i) remain unchanged.

2. The polypeptide or polypeptide complex according to claim 1, whereby the luxC polypeptide contains additionally at least one of the following amino acid substitutions of N27T, L132I, T216I, Y248N, K345N, T445K and/or the luxD polypeptide contains additionally at least one of the following amino acid substitutions of N3K, I90T, N165K, V251A, D274N or a variant of the polypeptide having at least 90 % identity therewith, whereby the additional amino acid substitutions identified in claim 1 or above remain unchanged.

3. The polypeptide or polypeptide complex according to claim 1 or 2, wherein the luxC polypeptide of SEQ ID NO. 2 contains additionally the amino acid substitution of E74N or in the luxC polypeptide of SEQ ID NO. 4 the additional amino acid substitutions of T10N and D74N.

4. A polypeptide complex containing a luxC polypeptide according to any one of claims 1 to 3, a luxD polypeptide according to any one of claims 1 to 3 and, additionally, a luxE polypeptide, preferably the luxE polypeptide of SEQ ID NO. 10.

5. A polypeptide composition comprising at least one of the polypeptide or polypeptide complex according to any one of claims 1 to 4 and, additionally, a luxA and luxB polypeptide, in particular, the luxA polypeptide of SEQ ID NO. 12 or SEQ ID NO. 23 and the luxB polypeptide of SEQ ID NO. 14 or SEQ ID NO. 25.

6. The polypeptide complex according to claims 1 to 4 or the polypeptide complex composition according to claim 5 comprising additionally a frp polypeptide of SEQ ID NO. 16 or of SEQ ID NO. 27.

7. An isolated nucleic acid molecule comprising a nucleic acid sequence encoding at least one of the polypeptides of claims 1 to 3.

8. The isolated nucleic acid molecule according to claim 7, wherein the nucleic acid molecule comprises at least one of the sequence of SEQ ID NO. 18 encoding the luxC polypeptide and of the sequence of SEQ ID NO. 20 encoding the luxD polypeptide.

9. The isolated nucleic acid molecule comprising the luxC gene with the luxD gene encoding the luxC polypeptide and luxD polypeptide of any one of claims 1 to 3 comprising additionally a luxE gene, preferably, the luxE gene of SEQ ID NO. 9.

10. The isolated nucleic acid molecule according to any one of claims 7 to 9 further comprising at least one of the luxA and luxB genes, in particular, the luxA gene of SEQ ID NO. 11 or the luxA gene of SEQ ID NO. 22, and the luxB gene of SEQ ID NO. 13 or the luxB gene of SEQ ID NO. 24.

11. The isolated nucleic acid molecule according to any one of claims 7 to 10 being the nucleic acid molecule of SEQ ID NO. 17 (operon).

12. An operon comprising at least one of the nucleic acid molecules according to any one of claims 7 to 11, in particular, being a luxCDE operon or a luxCDABE operon.

13. The operon according to claim 12 further comprising a flavin reductase gene, in particular, the frp gene of SEQ ID NO. 15 or SEQ ID NO. 26.

14. A vector, bacteriophage or virus comprising an operon according to any one of claims 12 or 13, or at least of the nucleic acid molecules according to any one of claim 7 to 11.

15. A cell, in particular, a bacterial cell, containing at least one of the isolated nucleic acid molecules according to any one of claims 7 to 11, the operon according to claims 12 or 13 or the vector, bacteriophage or virus according to claim 14.

16. A cell, in particular, a bacterial cell, containing at least the nucleic acid molecules according to claims 7 to 11, encoding the luxG polypeptide and the luxD polypeptide, optionally, the luxE polypeptide according to any one of claims 1 to 6 and, separately, a luxAB frp gene construct.

17. The cell, in particular, the bacterial cell according to any one of claims 15 or 16, wherein at least one nucleic acid molecule according to any one of claims 7 to 11, or the operon according to claims 12 or 13, is integrated within the chromosome of the cell, in particular, the bacterial cell.

18. A method of generation of bright autobioluminescent cells, in particular, bacterial cells, having improved lux operon activity comprising the step of introducing one isolated nucleic acid molecule according to any one of claims 7 to 11, the operon according to claims 12 or 13 or the vector, bacteriophage or virus according to claim 14 into cells, in particular, the bacterial cell, like *E. coli.*

19. A kit comprising at least one isolated nucleic acid molecule according to any one of claims 7 to 11, an operon according to claims 12 or 13 or a vector, bacteriophage or virus according to claim 14 and, optionally, introducing the at least one nucleic acid molecule, the operon or the vector into cells, in particular, bacterial cells.

20. The use of at least one nucleic acid molecule according to any one of claims 7 to 11, an operon according to claims 12 or 13, the vector, bacteriophage or virus according to claim 14, the cells according to claims 15 to 17, or a polypeptide according to any one of claims 1 to 6 in a process of bioluminescence imaging in living cells, preferably, for detecting and imaging bacterial cells, like single bacterial cells.

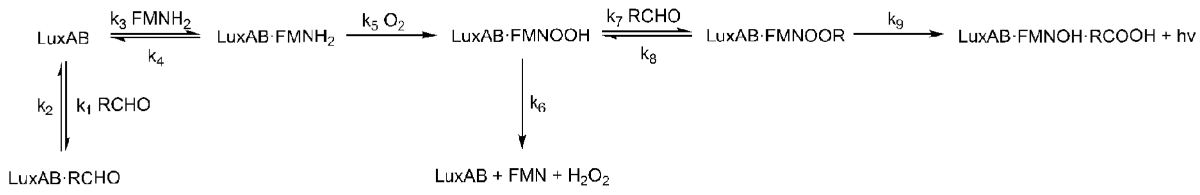


Figure 1

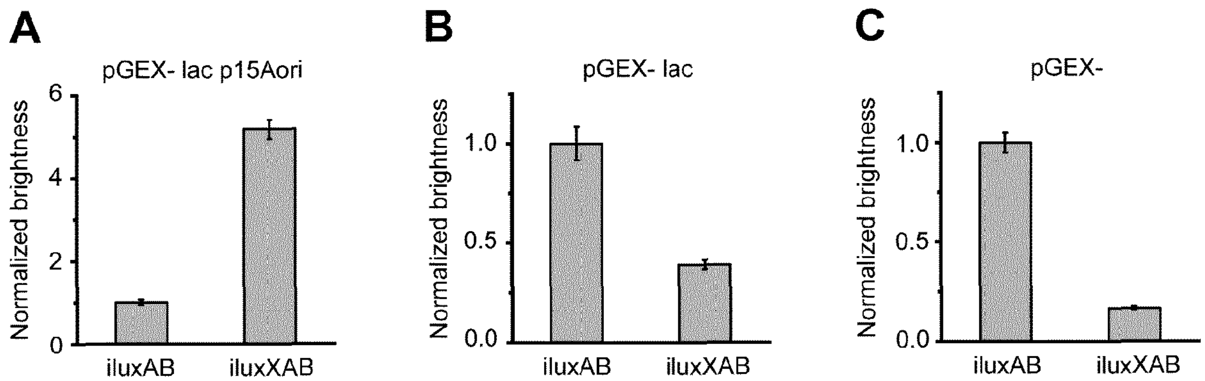


Figure 2

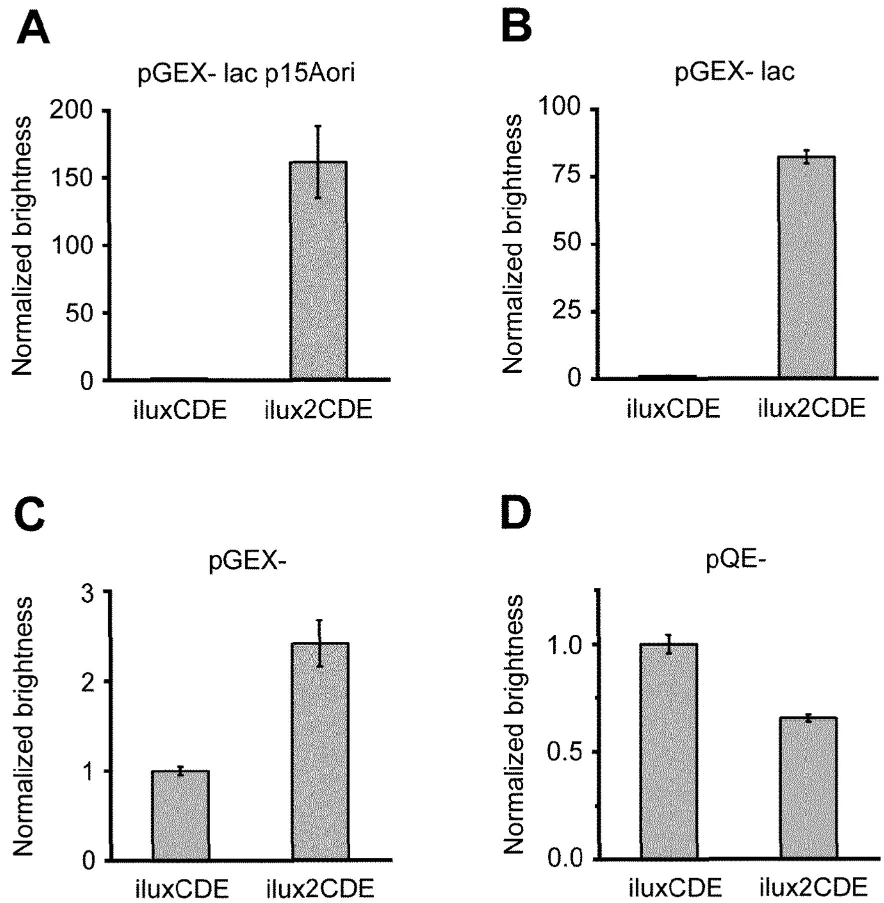


Figure 3

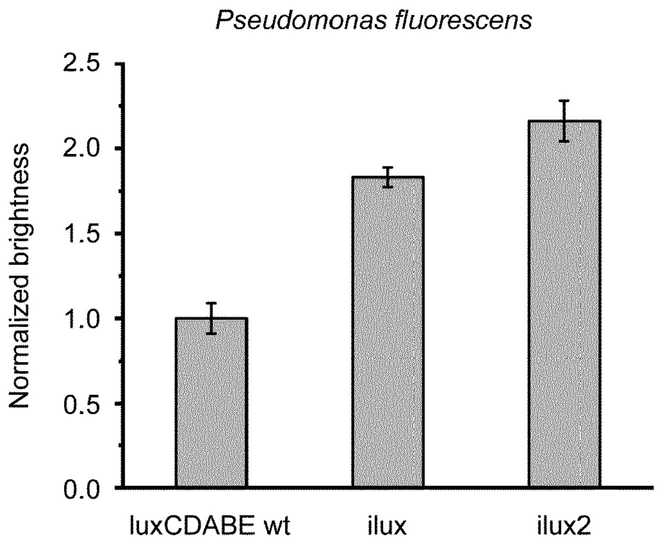


Figure 4

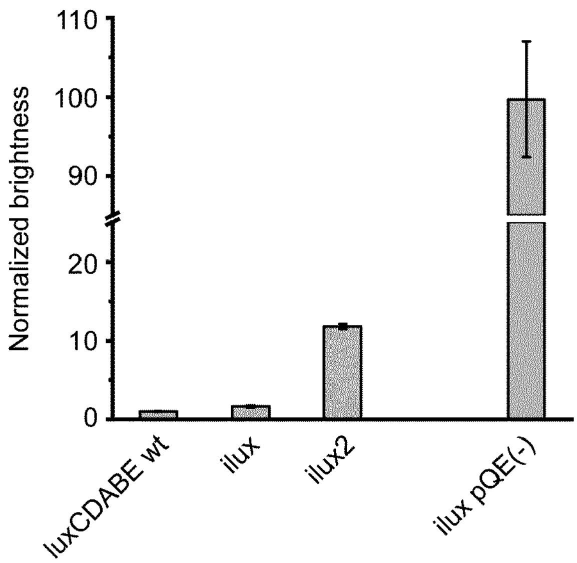


Figure 5

Europäisches Patentamt
European Patent Office
Office européen des brevets

# EUROPEAN SEARCH REPORT

Application Number
EP 21 20 9450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GenBank [Online]<br><br>8 October 2021 (2021-10-08), Castaneda-Alvarez C. ET AL: "Acyl transferase [Photorhabdus sp. UCH-936]", XP055926614, retrieved from EBI Database accession no. MCA6221101 | 1 | INV.<br>C12N9/02<br>C12N9/10<br>C12N15/63 |
| A | * the whole document * | 2-20 | |
| A | WO 2014/159825 A1 (BIOGLOW LLC [US]) 2 October 2014 (2014-10-02)<br>* abstract *<br>* page 2, line 22 - line 25 *<br>* page 5, line 5 - line 6 *<br>* pages 8-12; example 2; sequence 9 * | 1-20 | |
| A | US 2017/044505 A1 (KRICHEVSKY ALEXANDER [US]) 16 February 2017 (2017-02-16)<br>* abstract *<br>* page 1, paragraphs 10,11 *<br>* page 4, paragraph 72 *<br>* pages 13-14; sequence 10 * | 1-20 | TECHNICAL FIELDS SEARCHED (IPC)<br>C12N<br>C40B |
| A,D | C. GREGOR ET AL.: PNAS, vol. 115, 2018, page 962, XP002806665,<br>* abstract *<br>* page 963; table 1 * | 1-20 | |

The present search report has been drawn up for all claims

| Place of search | Date of completion of the search | Examiner |
|---|---|---|
| Munich | 1 June 2022 | Grötzinger, Thilo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO.

EP 21 20 9450

This annex lists the patent family members relating to the patent documents cited in the above-mentioned European search report. The members are as contained in the European Patent Office EDP file on
The European Patent Office is in no way liable for these particulars which are merely given for the purpose of information.

01-06-2022

| Patent document cited in search report | Publication date | Patent family member(s) | Publication date |
|---|---|---|---|
| WO 2014159825 A1 | 02-10-2014 | US 8747835 B1 | 10-06-2014 |
| | | US 2014273224 A1 | 18-09-2014 |
| | | WO 2014159825 A1 | 02-10-2014 |
| US 2017044505 A1 | 16-02-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **C. GREGOR et al.** *PNAS,* 2018, vol. 115, 962
- **GREGOR et al.** *PNAS,* 2018, vol. 115, 962
- **C.R. REISCH et al.** *Sci. Rep.,* 2015, vol. 5, 15096
- **J. HOFFMANN et al.** *PLoS One,* 2015, vol. 10, e1033248
- **G.J. MCKENZIE et al.** *BMC Microbiol,* 2006, vol. 6, 39
- **C. GREGOR et al.** *PNAS,* 2019, vol. 116, 26491
- **C. GREGOR.** *Methods Mol. Biol.,* 2020, vol. 2081, 43
- **W.A. FRANCISCO et al.** *Biochemistry,* 1998, vol. 37, 2596
- **R. TINIKUL et al.** Bioluminescence: Fundamentals and Applications in Biotechnology. Springer, 2014, vol. 3, 47
- **H.M. ABU-SOUD et al.** *J. Biol. Chem.,* 1993, vol. 268, 7699
- **Z.T. CAMPBELL et al.** *J. Biol. Chem.,* 2009, vol. 284, 8322
- **S. ULITZUR et al.** *PNAS,* 1978, vol. 75, 266
- **D. RIENDEAU et al.** *J. Biol. Chem.,* 1979, vol. 254, 7488
- **J.W. HASTINGS et al.** *J. Biol. Chem.,* 1963, vol. 238, 3100
- **J.W. HASTINGS et al.** *Biochemistry,* 1969, vol. 8, 4681